(19) Europäisches Patentamt / European Patent Office / Office européen des brevets

(11) **EP 2 611 439 B1**

(12) **EUROPÄISCHE PATENTSCHRIFT**

(45) Veröffentlichungstag und Bekanntmachung des Hinweises auf die Patenterteilung:
**21.02.2018 Patentblatt 2018/08**

(21) Anmeldenummer: **11746585.6**

(22) Anmeldetag: **26.08.2011**

(51) Int Cl.:
*A61K 31/202* (2006.01)      *A61K 31/513* (2006.01)
*A61P 43/00* (2006.01)      *A61N 5/10* (2006.01)

(86) Internationale Anmeldenummer:
**PCT/EP2011/064693**

(87) Internationale Veröffentlichungsnummer:
**WO 2012/028543 (08.03.2012 Gazette 2012/10)**

(54) **DIE PARENTERALE APPLIKATION VON FISCHÖL/DHA +EPA VOR BZW. MIT BEGINN DER CHEMOTHERAPIE**

COMPOSITION COMPRISING A COMBINATION OF DHA AND EPA FOR PARENTERAL ADMINISTRATION PRIOR TO COMMENCEMENT OF CHEMOTHERAPY

COMPOSITION COMPRENANT UNE COMBINAISON DE DHA ET D'EPA À ADMINISTRER PAR VOIE PARENTÉRALE AVANT LE DÉBUT D'UNE CHIMIOTHÉRAPIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.09.2010 US 379775 P**
**03.09.2010 EP 10175293**

(43) Veröffentlichungstag der Anmeldung:
**10.07.2013 Patentblatt 2013/28**

(73) Patentinhaber: **Fresenius Kabi Deutschland GmbH**
**61352 Bad Homburg v.d.H. (DE)**

(72) Erfinder:
• **SCHLOTZER, Ewald**
**61440 Oberursel (DE)**
• **KRAMPITZ, Barbara**
**60486 Frankfurt am Main (DE)**
• **SUCHNER, Ulrich**
**85435 Erding (DE)**

(74) Vertreter: **Fresenius Kabi Deutschland GmbH**
**Patent Department**
**Else-Kröner-Straße 1**
**61352 Bad Homburg (DE)**

(56) Entgegenhaltungen:
**WO-A1-01/49284      WO-A1-98/09621**
**US-A1- 2003 068 385**

• **TAKAGI K ET AL: "Perioperative supplementation of EPA reduces immunosuppression induced by postoperative chemoradiation therapy in patients with esophageal cancer.", NUTRITION (BURBANK, LOS ANGELES COUNTY, CALIF.), Bd. 17, Nr. 6, Juni 2001 (2001-06), Seiten 478-479, XP002614617, ISSN: 0899-9007**
• **Rote List Service: "Omegaven Fresenius", Fachinformation , April 2010 (2010-04), XP002664639, Gefunden im Internet: URL:http://www.fachinfo.de/data/fi/jsearch ?pra ep [gefunden am 2011-11-30]**

EP 2 611 439 B1

**Beschreibung**

Gebiet der Erfindung

Die vorliegende Erfindung bezieht sich auf die Verwendung einer Omega-3-Fettsäuren umfassenden Zusammensetzung zur Verabreichung an Krebspatienten vor dem Beginn eines Zyklus einer Chemotherapie oder Strahlentherapie. Durch die Gabe der Zusammensetzung wird die Wirksamkeit der Therapie verbessert. Nebenwirkungen der Therapie werden durch die Gabe der Therapie verhindert oder abgemildert.

Hintergrund der Erfindung

Krebserkrankungen stellen weltweit eine der häufigsten Todesursachen dar. Die häufigsten Krebserkrankungen bei Frauen sind das Mammakarzinom, Lungenkarzinom und das kolorektale Karzinom. Männer erkranken besonders häufig am Prostatakarzinom, Lungenkarzinom und am kolorektalen Karzinom (Jemal et al. 2009; CA Cancer J Clin; 59(4): 225-49). Allen Krebserkrankungen ist gemeinsam, dass es durch Veränderungen in der Zelle zu deren unkontrolliertem Wachstum kommt.
Eine Behandlung der Krebserkrankung erfolgt je nach Art und Lokalisation des Tumors sowie je nach Erkrankungsstadium durch chirurgische Entfernung des Tumors, Chemotherapie, Strahlentherapie, Immuntherapie oder weitere, sogenannte zielgerichtete Therapieformen, die zum Beispiel die Behandlung mit monoklonalen Antikörpern umfassen. Grundsätzlich können Chemotherapie und Strahlentherapie als alleinige Therapie angewandt werden. Oft werden jedoch, je nach Erkrankungsstadium des Patienten, mehrere Therapieformen kombiniert. Etwa wird ein Tumor häufig zunächst chirurgisch entfernt und danach eine Chemotherapie angewandt, um etwaige im Köper verbliebene Krebszellen abzutöten. Eine Strahlentherapie kann zum Beispiel eingesetzt werden, um zunächst eine Schrumpfung des Tumors zu erreichen, was die nachfolgende chirurgische Entfernung des Tumors erleichtert. Eine Kombination aus Chemotherapie und Strahlentherapie ("Radiochemotherapie") wird unter Anderem in der Behandlung von Tumoren des Rektums, des Gebärmutterhalses, der Lunge, der Brust, der Speiseröhre sowie bei Kopf-Hals-Tumoren eingesetzt.
Trotz verbesserter Therapiemöglichkeiten für Patienten mit Krebserkrankungen ist die Erkrankung in einer Vielzahl von Fällen nicht ursächlich heilbar. Die Therapie kann das Fortschreiten der Erkrankung hier unter Umständen lediglich verlangsamen (Jemal et al. 2009; CA Cancer J Clin; 59(4): 225-49).
Die zur Behandlung von Krebserkrankungen eingesetzten Chemotherapeutika und auch die häufig eingesetzte Strahlentherapie rufen bei den Patienten starke Nebenwirkungen hervor (Ladewski et al. 2003; J Clin Oncol. 21(20):3859-66). Dies ist dadurch zu erklären, dass die Wirkung von Chemotherapeutika und Strahlentherapie nicht auf die Krebszellen beschränkt ist. Es kommt zur Schädigung gesunder Zellen. Betroffen sind vor allem solche Zellen, die eine starke Teilungsaktivität aufwesen, wie etwa die Zellen der Schleimhäute. Beobachtet werden zum Beispiel Stomatitis, Mukositis, Erbrechen und Diarrhöe. Weitere Nebenwirkungen betreffen das blutbildende System, auch Nervenzellen können betroffen sein.
Die starken Nebenwirkungen, die üblicher Weise mit einer Krebstherapie einhergehen, belasten den Patienten und sind letztlich auch mitentscheidend dafür, in welchem Umfang oder in welcher Dosierung die Chemo- oder Strahlentherapie eingesetzt werden kann.
WO98/09621 beschreibt die zytotoxischen Effekte verschiedener Fettsäuren, unter anderem die von EPA und DHA, sowie deren lindernde Wirkung hinsichtlich der Chemotherapie-bedingten Nebenwirkungen einer Krebsbehandlung. WO98/09621 macht jedoch keine konkreten Angaben zum konkreten Dosierungsschema und insbesondere nicht zum Zeitpunkt, zu dem die Fettsäuren verabreicht werden sollen.
Die Supplementierung von Krebspatienten mit EPA im Zeitraum von einer Woche vor der Operation bis 2 Wochen nach der Operation zusätzlich zur Verabreichung einer postoperativen Chemotherapie hat möglicherweise positive Auswirkungen auf das Gesamtüberleben der Patienten (Nutrition Volume 17, Number 6, 2001, S. 478-479). Ein solches Dosierungsschema muss jedoch langfristig geplant werden und gestattet folglich keine kurzfristige und/oder spontane Intervention.
Es besteht also weiterhin ein Bedarf nach Zusammensetzungen und Verfahren zur Verhinderung oder Abschwächung des Auftretens von Nebenwirkungen bei Krebstherapien. Dadurch könnten die Verträglichkeit, Wirksamkeit und die Akzeptanz der Therapie durch die Patienten verbessert werden. Ferner besteht ein Bedarf nach Zusammensetzungen und Verfahren, um die Wirksamkeit von Chemo- oder Strahlentherapie zu erhöhen. Eine erhöhte Wirksamkeit ermöglicht die Verminderung der zur Therapie einzusetzenden Dosierungen und dadurch auch eine Verminderung der mit der Therapie einhergehenden Nebenwirkungen.

Zusammenfassende Beschreibung der Erfindung

Die Erfindung betrifft eine Zusammensetzung umfassend Omega-3-Fettsäuren. Die Zusammensetzung kann zur Verbesserung der Wirksamkeit einer Chemotherapie oder einer Strahlentherapie und / oder in der Vorbeugung oder Reduktion von durch die Chemotherapie oder die Strahlentherapie hervorgerufenen Nebenwirkungen in einem an Krebs erkrankten Patienten verwendet werden, wobei die Zusammensetzung dem Patienten vor Beginn eines Zyklus der Chemotherapie oder der Strahlentherapie zu verabreichen ist.

Abbildungen

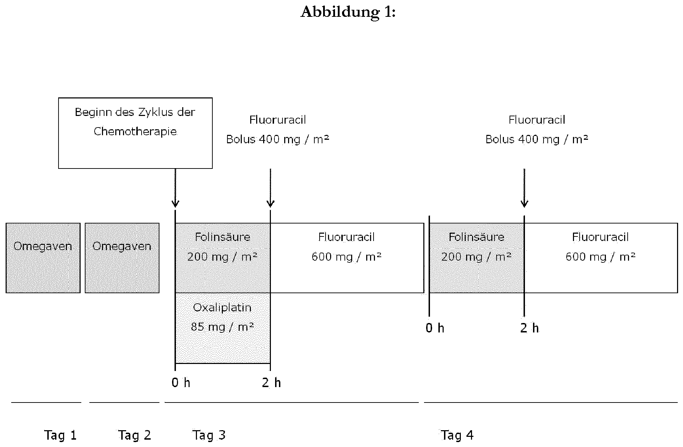

**Abbildung 1:** Applikationsschema zur Verabreichung einer Zusammensetzung umfassend EPA und DHA (Omegaven®) vor dem Beginn eines Zyklus einer Chemotherapie. Die Abbildung zeigt beispielhaft die konsekutive intravenöse Applikation von Omegaven® zwei Tage ("Tag 1") und einen Tag ("Tag 2") vor dem Beginn eines Zyklus ("Tag 3") einer 5-FU basierten adjuvanten Chemotherapie bei einem Patienten mit kolorektalem Karzinom. Das angewandte Chemotherapieschema ist das sogenannte FOLFOX-Schema. Bei FOLFOX handelt es sich um eine Kombinationstherapie aus den Medikamenten Folinsäure, Fluorouracil und Oxaliplatin. FOLFOX ist das am weitesten verbreitete Therapieschema zur Behandlung des Kolonkarzinoms. Die Dosierungsangabe der Medikamente der Chemotherapie erfolgt als mg / m$^2$ Körperoberfläche des Patienten. Die Gabe von Omegaven® erfolgt in einer Dosierung von 2 mL / kg Körpergewicht des Patienten und Tag. Die Darstellung der Tage und der verabreichten Zusammensetzung und Medikamente sind schematisch und die Breite der Kästchen korrespondiert nicht mit der Zeitdauer der Verabreichung.

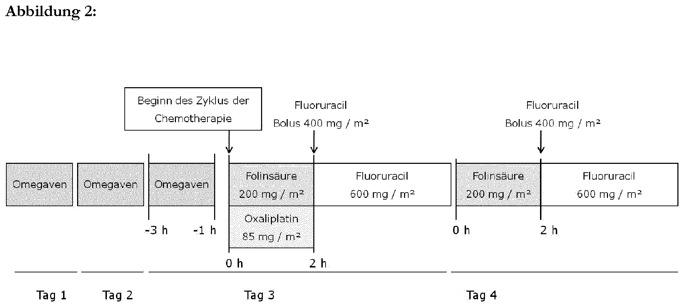

Abbildung 2: Applikationsschema zur Verabreichung einer Zusammensetzung umfassend EPA und DHA (Omegaven®) vor dem Beginn eines Zyklus einer Chemotherapie, wobei die Verabreichung auch noch 3 Stunden vor dem Beginn des Zyklus erfolgt. Die Abbildung zeigt beispielhaft die konsekutive intravenöse Applikation von Omegaven® zwei Tage ("Tag 1"), einen Tag ("Tag 2") und am Tag des Beginns ("Tag 3") eines Zyklus einer 5-FU basierten adjuvanten Chemotherapie nach dem FOLFOX-Schema bei einem Patienten mit kolorektalem Karzinom. Die Verabreichung am Tag des Beginns des Chemotherapiezyklus erfolgt 3 Stunden ("-3h") vor dem Beginn des Zyklus, und ist spätestens eine Stunde ("-1 h") vor dem Beginn des Zyklus beendet. Die Dosierungsangabe der Medikamente der Chemotherapie erfolgt als mg / m$^2$ Körperoberfläche des Patienten. Die Gabe von Omegaven® erfolgt in einer Dosierung von 2 mL / kg Körpergewicht des Patienten und Tag. Die Darstellung der Tage und der verabreichten Zusammensetzung und Medikamente sind schematisch und die Breite der Kästchen korrespondiert nicht mit der Zeitdauer der Verabreichung.

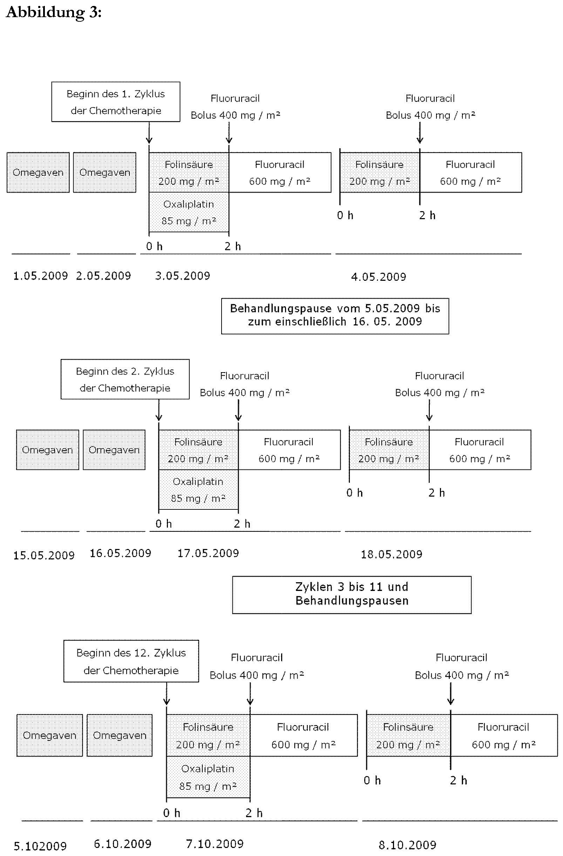

**Abbildung 3:** Applikationsschema zur Verabreichung einer Zusammensetzung umfassend EPA und DHA (Omegaven®) vor dem Beginn jedes Zyklus einer 12 Zyklen umfassenden Chemotherapie. Die Abbildung zeigt beispielhaft die konsekutive intravenöse Applikation von Omegaven® zwei Tage und einen Tag vor jedem Zyklus einer 12 Zyklen umfassenden 5-FU basierten adjuvanten Chemotherapie nach dem FOLFOX-Schema bei einem Patienten mit kolorektalem Karzinom. Die Verabreichung erfolgt jeweils vor dem Beginn eines Zyklus. Ein Zyklus der Chemotherapie umfasst zwei Tage. Der Zyklus wird gefolgt von einer mehrtägigen Behandlungspause, in der der Patient keine Chemotherapeutika erhält, und die bis zum Beginn des nächsten Zyklus der Chemotherapie reicht. Darauf folgt der zweite Zyklus der Chemotherapie. Omegaven® wird vor dem zweiten Zyklus, also während der Behandlungspause, verabreicht. Das Verabreichungsschema für Omegaven® wird für die folgenden Zyklen 3 bis 11 (nicht dargestellt) identisch durchgeführt. Omegaven® wird also zwei Tage und einen Tag vor dem Beginn des jeweiligen Zyklus der Chemotherapie verabreicht. Es sind prinzipiell aber auch Variationen im Verabreichungsschema möglich, solange die Verabreichung vor dem Beginn des Zyklus erfolgt, und solange die Verabreichung nicht in den dem jeweiligen Zyklus vorangehenden Zyklus fällt. Die Verabreichung vor dem zwölften (letzten) Zyklus der Chemotherapie erfolgt wie vorstehend für die Zyklen 1 bis 11 beschrieben. Die Darstellung der Tage und der verabreichten Zusammensetzung und Medikamente sind schematisch und die Breite der Kästchen korrespondiert nicht mit der Zeitdauer der Verabreichung.

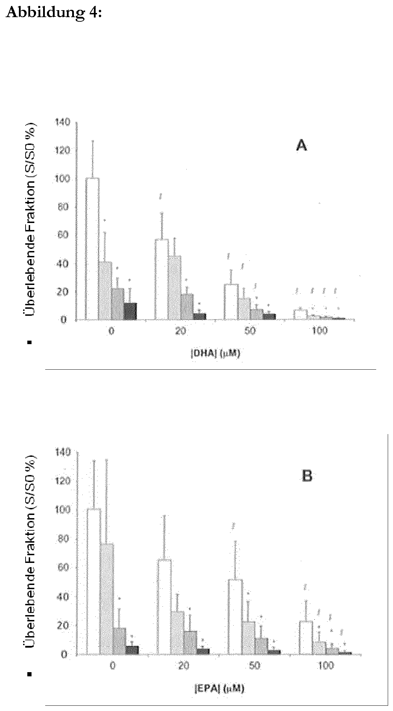

**Abbildung 4:** Einfluss von DHA und EPA auf das Überleben von HT-29 Zellen nach Bestrahlung. Die Abbildung zeigt das Überleben von HT-29 Zellen nach einer zweitägigen Vorbehandlung mit 20 $\mu$M bis 100 $\mu$M DHA (A) oder EPA (B) gefolgt von einer Bestrahlung mit 0 Gy (weiße Balken), 2 Gy (hellgraue Balken), 4 Gy (mittelgraue Balken) oder 6 Gy (schwarze Balken). Die Werte sind als Mittelwerte (%) $\pm$ Standartabweichung relativ zu den unbehandelten Kontrollen (6 pro Gruppe) angegeben. * P < 0,05 im Vergleich mit 0 Gy; $^{\S}$ P < 0,05 im Vergleich mit unbehandelter Kontrolle.

Eingehende Beschreibung der Erfindung

Die Erfindung wird durch die beigefügten Ansprüche definiert.

Definitionen:

Der Begriff **Verabreichung** umfasst die enterale und parenterale Verabreichung.

**Enterale Verabreichung** bezeichnet die Zufuhr über den Darm, zum Beispiel durch orale Aufnahme, oder durch transnasale, gastrale oder jejunale Sonden.

Unter **parenteraler Verabreichung** im Sinne der hier vorliegenden Erfindung wird eine Verabreichung unter Umgehung des Darms verstanden. Umfasst sind somit zum Beispiel die intravenöse Injektion oder Infusion sowie die intraarterielle Injektion oder Infusion. Als **intraarteriell** wird die Verabreichung in ein arterielles Blutgefäß verstanden, als **intravenös** die Verabreichung in ein venöses Blutgefäß. Mit Injektion wird die Verabreichung per Spritze umfasst. In der Regel erfolgt diese als Bolus. Eine kontinuierliche Injektion mittels Spritzenpumpen ist aber ebenso möglich. Mit Infusion wird die kontinuierliche Verabreichung der Zusammensetzung in ein Blutgefäß bezeichnet, die zum Beispiel über einen peripheren oder zentralen Venenkatheter erfolgen kann. Ebenso umfasst ist die **transdermale** Verabreichung (die Verabreichung über die Haut).

Unter **Chemotherapie** wird die medikamentöse Therapie von Krebserkrankungen verstanden. Eine Chemotherapie kann die Behandlung mit einem oder mehreren Medikamenten, sogenannten **Chemotherapeutika**, zur Therapie von Krebserkrankungen umfassen. Es kann eine Kombination von Medikamenten eingesetzt werden, die zeitgleich oder zeitlich versetzt verabreicht werden. Chemotherapeutika, die zur medikamentösen Therapie von Krebserkrankungen eingesetzt werden, werden zum Teil auch zur Behandlung anderer Erkrankungen, wie etwa schweren Autoimmunerkrankungen eingesetzt.

Bei **FOLFOX** handelt es sich um ein zur Chemotherapie des kolorektalen Karzinoms eingesetztes Chemotherapie-Schema, umfassend die Medikamente Oxaliplatin, Folinsäure (Leucovorin) und Fluoruracil (5-FU).

Bei **FOLFIRI** handelt es sich ebenfalls um ein zur Chemotherapie des kolorektalen Karzinoms eingesetztes Chemotherapie-Schema, umfassend die Medikamente Folinsäure (Leucovorin), Fluoruracil (5-FU) und Irinotecan.

**Strahlentherapie** umfasst die Anwendung von ionisierender Strahlung, zum Beispiel Gammastrahlung, Röntgenstrahlung und Elektronen. Ebenso umfasst ist die Anwendung von Neutronen, Protonen und schweren Ionen zur Behandlung von Krebspatienten. Eine Strahlentherapie kann beispielsweise in der Form der Teletherapie oder Brachytherapie erfolgen. Der Ausdruck "Verabreichung einer Bestrahlung" oder "Verabreichung der Bestrahlung" kann im Sinne der hier vorliegenden Erfindung gleichwertig mit dem Ausdrucken "bestrahlen" verwendet werden.

Unter einem **Zyklus** im Sinne der hier vorliegenden Erfindung wird ein Zeitraum verstanden, in dem einem Patienten eine Bestrahlung, ein Chemotherapeutikum, mehrere Chemotherapeutika oder eine Kombination davon verabreicht werden. Bei einmaliger Verabreichung der Bestrahlung, des Chemotherapeutikums, mehrerer Chemotherapeutika oder eine Kombination davon umfasst der Zyklus den Zeitraum vom Beginn der einmaligen Verabreichung bis zum Ende der einmaligen Verabreichung.

Wird eine Bestrahlung, ein Chemotherapeutikum, mehrere Chemotherapeutika oder eine Kombination an aufeinanderfolgenden Tagen verabreicht, so umfasst der Zyklus den Zeitraum vom Beginn der ersten Verabreichung bis zum Ende der letzten Verabreichung. Die Verabreichung der Bestrahlung, des Chemotherapeutikums, der Chemotherapeutika oder einer Kombination davon kann zeitgleich oder zeitlich versetzt erfolgen und kann einzeln oder in Kombination wiederholt werden. Erhält der Patient an einem auf eine Verabreichung einer Bestrahlung, eines Chemotherapeutikums, von Chemotherapeutika oder einer Kombination davon folgenden Tag weder Bestrahlung noch ein Chemotherapeutikum noch mehrere Chemotherapeutika, ist der Zyklus mit dem Ende der letzten Verabreichung beendet.

Auf den Zyklus folgt eine Behandlungspause. Diese ist dadurch gekennzeichnet, dass dem Patienten während der Behandlungspause keine Bestrahlung und keine Chemotherapie verabreicht werden.

Nach einer Behandlungspause können sich ein oder mehrere weitere Zyklen anschließen. Eine Chemotherapie oder Strahlentherapie kann also einen oder mehrere Zyklen umfassen.

Der **Beginn des ersten Zyklus** einer Chemotherapie oder einer Strahlentherapie kann der Zeitpunkt sein, ab dem ein Patient erstmals eine bestimmte Chemotherapie oder eine bestimmte Strahlentherapie erhält, also der Zeitpunkt, ab dem dem Patienten erstmals eine Bestrahlung, ein Chemotherapeutikum, mehrere Chemotherapeutika oder eine Kombination verabreicht werden. Der **Beginn eines Zyklus** ist der Zeitpunkt, zu dem die Verabreichung einer Bestrahlung, eines Chemotherapeutikum, mehrerer Chemotherapeutika oder eine Kombination davon beginnt. Werden mehr als ein Element ausgewählt aus der Gruppe aus Bestrahlung, ein Chemotherapeutikum, und mehrere Chemotherapeutika verabreicht, so beginnt der Zyklus mit dem Beginn der Verabreichung des Elements, das als erstes verabreicht wird, und endet mit dem Ende der Verabreichung des Elements, das als letztes verabreicht wird.

**Solide Tumoren** gemäß der hier vorliegenden Erfindung umfasst Tumoren, die nicht vom blutbildenden System abgeleitet sind. Solide Tumoren sind feste, zunächst örtlich begrenzte Tumoren. Der Begriff umfasst auch Absiedlungen des Tumors in andere Organe, sogenannte Metastasen. Solide Tumoren können benigne oder maligne sein. Tritt ein maligner solider Tumor in einem Patienten auf, so ist der Patient an Krebs erkrankt. Umfasst sind alle Arten solider Tumoren. Bevorzugte solide Tumoren sind das kolorektale Karzinom, das Mammakarzinom, das Pankreaskarzinom, das Leberkarzinom, das Lungenkarzinom, und das Magenkarzinom. Ganz besonders bevorzugt ist das klorektale Karzinom.

Der Begriff **nicht-solide Tumoren** gemäß der hier vorliegenden Erfindung umfasst Krebsarten des blutbildenden Systems. Der Begriff umfasst auch Absiedlungen in andere Organe, sogenannte Metastasen. Zu den nicht-soliden Tumoren gehören die Leukämien, umfassend akute myeloische Leukämie (AML, auch als akute nicht-lymphatische Leukämie (ANLL) bezeichnet), chronische myeloische Leukämie (CML), akute lymphatische Leukämie (ALL), chronische lymphatische Leukämie (CLL) und Lymphome, umfassend Hodgkin-Lymphome und Non-Hodgkin-Lymphome.

Der Begriff **kolorektales Karzinom** umfasst Karzinome des Kolons und / oder des Rektums. Auch Absiedlungen des kolorektalen Karzinoms in andere Organe, sogenannte Metastasen, sind umfasst.

Unter **Verbesserung der Wirksamkeit** im Sinne der hier vorliegenden Erfindung wird eine Erhöhung der Wirksamkeit einer Chemotherapie oder Strahlentherapie verstanden. Bei gleicher Dosierung übt die Chemotherapie oder Strahlentherapie bei verbesserter Wirksamkeit eine stärkere Wirkung auf den Tumor aus. Dies kann dazu führen, dass Parameter wie das Tumorwachstum, das Tumorvolumen, oder die Tumorzellabsiedlung, oder eine Kombination davon günstig beeinflusst werden. Eine Verbesserung der Wirksamkeit einer Chemotherapie oder Strahlentherapie kann zum Beispiel zu einer verstärkten Abnahme des Tumorvolumens führen. Ebenso kann eine Verbesserung der Wirksamkeit dazu führen, dass das Tumorvolumen über längere Zeit konstant bleibt, oder der Tumor weniger schnell oder weniger stark wächst, als dies ohne die Verbesserung der Wirksamkeit der Fall wäre. Eine Erhöhung der Wirksamkeit einer Chemotherapie oder Strahlentherapie kann dazu führen, dass die Dosierung der Therapie niedriger gewählt werden kann, und dennoch die gleiche Wirkung auf den Tumor erzielt werden kann, die die höhere Dosierung ohne eine Verbesserung ihrer Wirksamkeit auf den Tumor hat. Eine erhöhte Wirksamkeit kann durch erhöhte Chemosensitivität der Zellen hervorgerufen werden.

Der Begriff **Chemosensitivität** bezeichnet in der Medizin die Empfindlichkeit von Krebszellen gegenüber wachstumshemmenden Zytostatika. Die Chemosensitivität von Krebszellen entscheidet häufig mit über den Erfolg der Chemotherapie.

Unter **Vorbeugung von Nebenwirkungen** im Sinne der hier vorliegenden Erfindung wird ein Verhindern des Auftretens einer oder mehrerer mit einer Behandlung typischerweise einhergehenden Nebenwirkungen verstanden. Unter der **Reduktion von Nebenwirkungen** wird die Abmilderung der Nebenwirkungen verstanden, also ein weniger stark ausgeprägtes Auftreten oder ein zeitlich verkürztes Auftreten der Nebenwirkung. Dem Fachmann sind die Nebenwirkungen, die mit einer Behandlung einher gehen können, und ihre Ausprägungsformen bekannt.

**Nebenwirkungen** sind Wirkungen, die neben der beabsichtigten eigentlichen Wirkung eines Medikaments oder einer Behandlungsform auftreten. Nebenwirkungen werden auch als unerwünschte Arzneimittelwirkungen bezeichnet.

Zu den Nebenwirkungen der Chemotherapie zur Behandlung von Krebspatienten zählen unter Anderem: gastrointestinale Nebenwirkungen (wie zum Beispiel Trockenheit des Mundes, Entzündung des Mundes, entzündliche Veränderungen der Schleimhaut (Mucositis) im Magen-Darm Trakt, Diarrhöe), hämatologische Nebenwirkungen (wie zum Beispiel Anämie, Thrombopenie, Neutropenie, Leukopenie, Myelosuppression, Störung der körpereigenen Immunabwehr, Störung der Blutgerinnung), Reduktion des Lebergewichts, neurotoxische Nebenwirkungen (zum Beispiel Schädigung der Nerven, Störung der Berührungsempfindlichkeit oder Sensibilität), das Herz betreffende Nebenwirkungen wie zum Beispiel Erkrankungen des Herzmuskels (Kardiomyopathie), inflammatorischen Nebenwirkungen, Gewichtsverlust, eingeschränkte Funktion der Immunabwehr, Haarverlust, Müdigkeit, Übelkeit, Erbrechen, oder eine Kombination davon.

Zu den bei Strahlentherapie beobachteten Nebenwirkungen gehören unter Anderem: Müdigkeit; Appetitlosigkeit, Abgeschlagenheit, Kopfschmerzen, Übelkeit, Erbrechen, Durchfall, Schädigung der Schleimhaut von Mund und Rachen, Schädigung der Schleimhaut des Verdauungstrakt, Schädigung der Blase.

**Omega-3-Fettsäuren** sind mehrfach ungesättigte Fettsäuren, wobei die letzte Doppelbindung der Fettsäure in der Omega-3 Position, also in der von dem Carboxyl-Ende aus gesehen drittletzten C-C-Bindung vorliegt. Die in der erfindungsgemäßen Zusammensetzung enthaltenen **Omega-3-Fettsäuren** können pflanzlichen oder tierischen Ursprungs sein, beispielsweise können die Fettsäuren aus Algen oder aus Fisch gewonnen werden. Bevorzugt sind **langkettige** und **überlangkettige** Omega-3-Fettsäuren. Besonders bevorzug sind **Omega-3-Fettsäuren,** die ausgewählt sind aus der Gruppe bestehend aus Eicosapentaensäure (EPA) und Docosahexaensäure (DHA), oder einer Kombination davon.

**Langkettige Omega-3-Fettsäuren** haben eine Kettenlänge von 12-17 Kohlenstoffatomen. **Überlangkettige Omega-3-Fettsäuren** (very long chain fatty acids, VLCFA) haben eine Kettenlänge von 18-26 Kohlenstoffatomen. Beispiele für VLCFAs sind Linolensäure, Eicosapentaensäure und Docosahexaensäure.

**Eicosapentaensäure** (EPA) oder (5Z,8Z,11Z,14Z,17Z)-Eicosa-5,8,11,14,17-pentaensäure ist eine mehrfach ungesättigte Fettsäure aus der Klasse der Omega-3-Fettsäuren mit der Summenformel $C_{20}H_{30}O_2$.

**Docosahexaensäure** (DHA) oder (4Z,7Z,10Z,13Z,16Z,19Z)-Docosa-4,7,10,13,16,19-hexaensäure ist eine mehrfach ungesättigte Fettsäure aus der Klasse der Omega-3-Fettsäuren mit der Summenformel $C_{22}H_{32}O_2$.

Unter **Fischöl** wird aus Fisch gewonnenes Öl verstanden, das Omega-3-Fettsäuren enthält. Das Fischöl kann aus Seefischen, zum Beispiel aus Hochseefischen gewonnen werden. Als **hochaufgereinigtes Fischöl** wird Fischöl bezeichnet, das zur parenteralen Verabreichung beim Menschen geeignet ist.

**Mittelkettige Triglyceride** (medium chain triglycerides, MCT) sind Triglyceride, die Fettsäurereste mittlerer Länge (einer Länge von 6 bis 12 C-Atomen) umfassen. Beispiele für MCT sind Capronsäure (C6), Caprylsäure (C8), Caprinsäure (C10), und Laurinsäure (C12), oder eine Kombination davon.

Unter **Eisen** im Sinne der hier vorliegenden Erfindung wird ein zur parenteralen Applikation geeignetes Eisensalz verstanden. Ferner werden auch hochmolekulare Eisenverbindungen, bestehend aus einem polymeren Eisen(III)-Oxid-Hydroxid-Kern verbunden mit einer Kohlenhydrathülle umfasst. Beispiele sind Eisencitrat, Eisendextran, Eisensulfat, Eisencarboxymaltose, Eisen(II)-chlorid, Eisenhydrogenaspartat, Eisen(II)-iodid, Eisenoxid, Eisen(III)-phosphat, Eisen(III)-natrium-gluconat-Komplex, Eisen-Sucrose Komplex sowie Eisen Saccharat Komplex.

Unter einer **konsekutiven Verabreichung** wird die Verabreichung an zwei oder mehreren aufeinanderfolgenden Tagen verstanden. Die konsekutive Verabreichung kann zum Beispiel an 2, 3, oder 4 aufeinanderfolgenden Tagen erfolgen. Dabei können zwischen zwei aufeinanderfolgenden Verabreichungen 24 Stunden, aber auch mehr oder weniger als 24 Stunden liegen. Zwischen zwei aufeinanderfolgenden Verabreichungen können 0 bis 48 Stunden liegen, zum Beispiel 5 bis 42 Stunden, 10 bis 38 Stunden oder 12 bis 36 Stunden. Zum Beispiel kann die Verabreichung an einem Tag morgens erfolgen, aber am darauffolgenden Tag mittags oder abends. bevorzugt ist, dass zwischen zwei aufeinanderfolgenden Verabreichungen 20 bis 28 Stunden, besonders bevorzugt 22 bis 26, am meisten bevorzugt 24 Stunden liegen Ebenso kann die Verabreichung zum Beispiel an einem Tag abends erfolgen und am darauffolgenden Tag morgens oder mittags. Eine konsekutive Verabreichung an mehreren aufeinanderfolgenden Tagen schließt eine mehrmalige Verabreichung am selben Tag nicht aus. Die Zusammensetzung kann einmal, zweimal, dreimal, viermal oder fünfmal täglich verabreicht werden.

Die vorliegende Erfindung basiert auf der Erkenntnis, dass eine Omega-3-Fettsäuren umfassende Zusammensetzung zur Verbesserung der Wirksamkeit einer Chemotherapie oder einer Strahlentherapie bei einem an Krebs erkrankten Patienten verwendet werden kann. Ebenso kann die Zusammensetzung zur Vorbeugung oder Reduktion von durch die Chemotherapie oder die Strahlentherapie hervorgerufenen Nebenwirkungen in einem an Krebs erkrankten Patienten eingesetzt werden.

Die Zusammensetzung umfasst zumindest die Omega-3-Fettsäuren EPA und DHA. Die Zusammensetzung kann weitere Omega-3-Fettsäuren enthalten. Ebenfalls bevorzugt ist eine Zusammensetzung umfassend langkettige oder überlangkettige Omega-3-Fettsäuren. Die Zusammensetzung kann eine oder mehrere verschiedene **Omega-3-Fettsäuren** umfassen. Wird von "Omega-3-Fettsäuren" im Plural gesprochen, können eine oder mehrere Omega-3-Fettsäuren umfasst sein, solange nichts anderes bestimmt wird. Wird von "Omega-3-Fettsäuren" im Singular gesprochen, können eine oder mehrere Omega-3-Fettsäuren umfasst sein, solange nichts anderes bestimmt wird.

Die Zusammensetzung kann weitere bevorzugte Zusatzstoffe, wie MCTs, Eisen, oder eine Kombination davon umfassen.

Die Zusammensetzung kann eine Emulsion sein.

Die Zusammensetzung wird parenteral und am meisten bevorzugt intravenös verabreicht.

Die Erfinder haben überraschend herausgefunden, dass die Wirksamkeit der Chemotherapie oder Strahlentherapie verbessert wird, wenn eine Omega-3-Fettsäuren wie EPA und DHA umfassende Zusammensetzung vor dem Beginn eines Zyklus der Therapie verabreicht wird. Eine zusätzliche Verabreichung während des Zyklus der Therapie oder nach Beendigung des Zyklus ist ebenfalls möglich. Die Wirksamkeit der Chemotherapie oder Strahlentherapie wird besonders dann verbessert, wenn die Zusammensetzung parenteral verabreicht wird. Die Verbesserung der Wirksamkeit entfaltet sich an Krebszellen.

Ebenso haben die Erfinder herausgefunden, dass eine Verabreichung der Zusammensetzung vor dem Beginn eines Zyklus der Therapie überraschend auch zur Vorbeugung oder Reduktion von durch die Chemotherapie oder die Strahlentherapie hervorgerufenen Nebenwirkungen führt. Eine besonders wirksame Vorbeugung oder Reduktion von durch die Chemotherapie oder die Strahlentherapie hervorgerufenen Nebenwirkungen erfolgt bei parenteraler Verabreichung der erfindungsgemäßen Zusammensetzung.

Der genaue der Erfindung zugrunde liegende Mechanismus ist nicht bekannt. Jedoch ist es wahrscheinlich, dass durch die Verabreichung, besonders durch die parenterale, zum Beispiel intravenöse, Verabreichung von Omega-3-Fettsäuren vor dem Beginn eines Zyklus einer Chemotherapie oder Strahlentherapie diese besser in die Zellmembranen inkorporiert werden können. Es liegen bei Beginn des Zyklus dann bereits Omega-3-Fettsäuren, bevorzugt von EPA und / oder DHA, in der Zellmembran gesunder Zellen vor, wo sie der Toxizität der Chemotherapeutika oder der Bestrahlung entgegenwirken und somit deren Nebenwirkungen verhindern bzw. reduzieren können. Eine Wirkungsabschwächung des Chemotherapeutikums oder der Strahlentherapie an der Tumorzelle wird nicht beobachtet. Die protektive Wirkung der Zusammensetzung entfaltet sich an den gesunden Zellen.

Die Erfinder haben herausgefunden, dass durch die Verabreichung der Zusammensetzung vor dem Beginn eines Zyklus einer Chemotherapie oder einer Strahlentherapie vorteilhafter Weise bereits Omega-3-Fettsäuren wie EPA und / oder DHA aus der Zusammensetzung im Körper zur Verfügung stehen und bereits ein Einbau in Zellmembranen erfolgt ist.

Durch die parenterale Verabreichung kann in vorteilhafter Weise eine hohe Dosierung an Omega-3-Fettsäuren wie EPA und / oder DHA verabreicht werden. Durch die intravenöse Verabreichung stehen die Omega-3-Fettsäuren schnell zum Einbau in die Zellmembran zur Verfügung, es kommt nicht zu Verlusten bei der intestinalen Resorption. Die erfindungsgemäße Zusammensetzung entfaltet ihre Wirksamkeit in Bezug auf die Vorbeugung oder Verminderung von Nebenwirkungen und die Verbesserung der Wirksamkeit einer Chemotherapie oder Strahlentherapie daher besonders schnell, und auch schon bei geringen Mengen. Erfindungsgemäß wird die Zusammensetzung erst 48 Stunden bis 24 Stunden vor Beginn eines Zyklus der Chemotherapie oder der Strahlentherapie verabreicht. Sogar wenn zum Beispiel die Zusammensetzung erst drei Stunden vor Beginn eines Zyklus verabreicht wird, ist die volle Wirksamkeit der Zusammensetzung durch die schnelle und direkte Bereitstellung gewährleistet. Es ist keine langwierige Supplementierung über mehrere Wochen erforderlich. Allerdings kann die konsekutive Gabe an zwei oder mehreren aufeinanderfolgenden Tagen den Einbau von Omega-3-Fettsäuren, wie DHA und EPA, in die Zellmembranen verstärken. Die Zusammensetzung kann zusätzlich zu der Gabe vor dem Beginn eines Zyklus auch während und / oder nach einem Zyklus verabreicht werden.

In vorteilhafter Wiese liegen Omega-3-Fettsäuren, wie EPA und DHA, bei Verabreichung der erfindungsgemäßen Zusammensetzung bereits vor dem Beginn eines Zyklus einer Chemotherapie oder einer Strahlentherapie vor, im Gegensatz zu aus dem Stand der Technik bekannten Zusammensetzungen, die mit Beginn oder nach Beginn einer Chemotherapie oder Strahlentherapie zu verabreichen sind.

Die Erfindung betrifft eine Zusammensetzung, die Omega-3-Fettsäuren, wie EPA und /oder DHA, enthält und zur Verwendung in der Verbesserung der Wirksamkeit einer Chemotherapie oder einer Strahlentherapie und / oder in der Vorbeugung oder Reduktion von durch die Chemotherapie oder die Strahlentherapie hervorgerufenen Nebenwirkungen in einem an Krebs erkrankten Patienten eingesetzt wird.

Die erfindungsgemäße Zusammensetzung wird dem Patienten vor Beginn eines Zyklus der Chemotherapie oder der Strahlentherapie verabreicht.

Die Zusammensetzung kann zusätzlich zur Verabreichung vor dem Beginn eines Zyklus auch während des Zyklus oder nach dem Zyklus erfolgen.

Die Verabreichung kann kontinuierlich oder intermittierend erfolgen.

Die Verabreichung kann parenteral erfolgen. Die parenterale Verabreichung erfolgt vorzugsweise intravenös. Die intravenöse Verabreichung kann als kontinuierliche Infusion oder als Bolusgabe erfolgen.

Die Zusammensetzung kann so verabreicht werden, dass eine intravenöse Verabreichung 0,05 mL bis 5,0 mL pro kg Körpergewicht und Stunde beträgt oder nicht überschreitet. Die intravenöse Verabreichung kann beispielsweise 0,1 mL bis 5,0 mL, 0,5 mL bis 5,0 mL, 1,0 mL bis 5,0 mL, 1,5 mL bis 5,0 mL, 2,0 mL bis 5,0 mL, 2,5 mL bis 5,0 mL, 3,0 mL bis 5,0 mL, 3,5 mL bis 5,0 mL, 4,0 mL bis 5,0 mL oder 4,5 mL bis 5,0 mL pro kg Körpergewicht und Stunde betragen oder nicht überschreiten. Die intravenöse Verabreichung kann 5,0 mL bis 0,05 mL, 4,5 mL bis 0,05 mL, 4,0 mL bis 0,05 mL, 3,5 mL bis 0,05 mL, 3,0 mL bis 0,05 mL, 2,5 mL bis 0,05 mL, 2,0 mL bis 0,05 mL, 1,5 mL bis 0,05 mL, 1,0 mL bis 0,05 mL oder 0,5 mL bis 0,05 mL pro kg Körpergewicht und Stunde betragen oder nicht überschreiten.

Vorzugsweise erfolgt die intravenöse Verabreichung so, dass die Verabreichung 0,5 mL bis 3,5 mL pro kg Körpergewicht und Stunde, bevorzugter 1,0 mL bis 3,0 mL, 1,5 mL bis 2,5 mL, am meisten bevorzugt 2,0 mL pro kg Körpergewicht und Stunde beträgt oder nicht überschreitet. Ebenfalls bevorzugt ist eine intravenöse Verabreichung, die 0,3 bis 0,5 mL pro kg Körpergewicht und Stunde beträgt oder nicht überschreitet.

Die erfindungsgemäße Zusammensetzung kann dem Patienten vor Beginn des ersten Zyklus der Chemotherapie oder der Strahlentherapie verabreicht werden. In einer bevorzugten Ausführungsform wird die erfindungsgemäße Zusammensetzung vor mehreren Zyklen, jeweils vor dem Beginn eines Zyklus der Chemotherapie oder der Strahlentherapie, verabreicht. Besonders bevorzugt ist es, die Zusammensetzung vor jedem der Zyklen zu verabreichen.

Die Verwendung der erfindungsgemäßen Zusammensetzung zur Verbesserung der Wirksamkeit einer Chemotherapie oder einer Strahlentherapie und / oder zur Vorbeugung oder Reduktion von durch die Chemotherapie oder die Strahlentherapie hervorgerufener Nebenwirkungen ist prinzipiell nicht auf bestimmte Nebenwirkungen beschränkt.

Die erfindungsgemäße Zusammensetzung wird zur Verbesserung der Wirksamkeit einer Chemotherapie oder einer Strahlentherapie und / oder Vorbeugung oder Reduktion von durch die Chemotherapie oder die Strahlentherapie hervorgerufenen Nebenwirkungen verwendet, wobei die Nebenwirkungen bevorzugt ausgewählt sind aus der Gruppe bestehend aus gastrointestinalen Nebenwirkungen, hämatologischen Nebenwirkungen, Reduktion des Lebergewichts, neurotoxischen Nebenwirkungen, das Herz betreffende Nebenwirkungen, inflammatorischen Nebenwirkungen, Gewichtsverlust, eingeschränkter Funktion der Immunabwehr, Reduktion von Entzündungen oder einer Kombination davon.

Ganz besonders bevorzugt wird die erfindungsgemäße Zusammensetzung zur Vorbeugung oder Reduktion von Nebenwirkungen verwendet, die bei der Chemotherapie oder Strahlentherapie eines kolorektalen Karzinoms auftreten.

Die erfindungsgemäße Zusammensetzung zur Verwendung in der Verbesserung der Wirksamkeit einer Chemotherapie oder einer Strahlentherapie und / oder in der Vorbeugung oder Reduktion von durch die Chemotherapie oder die Strahlentherapie hervorgerufenen Nebenwirkungen in einem an Krebs erkrankten Patienten ist prinzipiell nicht auf bestimmte Krebserkrankungen beschränkt.

Die Zusammensetzung kann bei einer Erkrankung an soliden oder nicht-soliden Tumoren verabreicht werden.

Bevorzugte solide Tumoren sind ausgewählt aus der Gruppe bestehend aus kolorektales Karzinom, Mammakarzinom, Pankreaskarzinom, Leberkarzinom, Lungenkarzinom, und Magenkarzinom. Ganz besonders bevorzugt ist die Zusammensetzung zur Verwendung bei einem am kolorektalen Karzinom erkrankten Patienten.

Die erfindungsgemäße Zusammensetzung kann bei jeder Form der Chemotherapie verabreicht werden.

In der Chemotherapie kann ein Chemotherapeutikum verwendet werden, das ausgewählt ist aus der Gruppe bestehend aus 5-Fluorouracil, Gemcitabin, Doxorubicin, Paclitaxel, Mitomycin, Cyclophosphamid, Epirubicin, Arabinosylcytosin, Tamoxifen, Irinotecan, Oxaliplatin, Folinsäure, Cisplatin, Taxane, Vinca Alkaloide, Epipodophyllotoxine, synthetische Alkaloide, Cytarabine, Nitrosurea, Dacarbazine, Fludarabine, Ifosfamid, Mytomicin C, Tamoxifen oder einer Kombination davon. Die Chemotherapie kann auch andere und /oder weitere Chemotherapeutika als die vorstehend genannten umfassen.

Insbesondere kann die Zusammensetzung auch verwendet werden, wenn der Patient neben der Chemotherapie oder der Strahlentherapie weitere Medikamente und / oder enterale oder parenterale Ernährung erhält.

Bevorzugt wird die erfindungsgemäße Zusammensetzung zur Verwendung in der Verbesserung der Wirksamkeit einer Chemotherapie oder einer Strahlentherapie und / oder in der Vorbeugung oder Reduktion von durch die Chemotherapie oder die Strahlentherapie hervorgerufenen Nebenwirkungen bei einer 5-Fluorouracil (5-FU) umfassenden Chemotherapie verabreicht, vorzugsweise wobei die Chemotherapie FOLFOX oder FOLFIRI ist.

Die Strahlentherapie kann ausgewählt sein aus der Gruppe bestehend aus Teletherapie und Brachytherapie.

Die erfindungsgemäße Zusammensetzung kann auch verwendet werden, wenn der Patient Chemotherapie und Strahlentherapie erhält. Beginnt der Zyklus der Chemotherapie und der Strahlentherapie nicht zum gleichen Zeitpunkt, ist die erfindungsgemäße Zusammensetzung bevorzugt vor demjenigen der beiden Zyklen zu verabreichen, der zu dem früheren Zeitpunkt beginnt.

Die erfindungsgemäße Zusammensetzung kann 0,5 g / 100 mL bis 10,0 g / 100 mL EPA umfassen. Die Zusammensetzung kann 1 g / 100 mL bis 10,0 g /100 mL EPA, 1,5 g / 100 mL bis 10,0 g / 100 mL EPA, 2 g / 100 mL bis 10,0 g / 100 mL EPA, 2,5 g / 100 mL bis 10,0 g / 100 mL EPA, 3 g / 100 mL bis 10,0 g / 100 mL EPA, 3,5 g / 100 mL bis 10,0 g / 100 mL EPA, 4 g / 100 mL bis 10,0 g / 100 mL EPA, 4,5 g / 100 mL bis 10,0 g / 100 mL EPA, 5 g / 100 mL bis 10,0 g / 100 mL EPA, 5,5 g / 100 mL bis 10,0 g / 100 mL EPA, 6 g / 100 mL bis 10,0 g / 100 mL EPA, 6,5 g / 100 mL bis 10,0 g / 100 mL EPA, 7 g / 100 mL bis 10,0 g / 100 mL EPA, 7,5 g / 100 mL bis 10,0 g / 100 mL EPA, 8 g / 100 mL bis 10,0 g / 100 mL EPA, 8,5 g / 100 mL bis 10,0 g /100 mL EPA, 9 g / 100 mL bis 10,0 g /100 mL EPA oder 9,5 g / 100 mL bis 10,0 g /100 mL EPA umfassen.

Die Zusammensetzung kann 0,5 g / 100 mL bis 9,5 g /100 mL EPA, 0,5 g / 100 mL bis 9 g / 100 mL EPA, 0,5 g / 100 mL bis 8,5 g / 100 mL EPA, 0,5 g / 100 mL bis 8 g / 100 mL EPA, 0,5 g / 100 mL bis 7,5 g / 100 mL EPA, 0,5 g / 100 mL bis 7 g / 100 mL EPA, 0,5 g / 100 mL bis 6,5 g / 100 mL EPA, 0,5 g / 100 mL bis 6 g / 100 mL EPA, 0,5 g / 100 mL bis 5,5 g / 100 mL EPA, 0,5 g / 100 mL bis 5 g / 100 mL EPA, 0,5 g / 100 mL bis 4,5 g / 100 mL EPA, 0,5 g / 100 mL bis 4 g /100 mL EPA, 0,5 g / 100 mL bis 3,5 g /100 mL EPA, 0,5 g / 100 mL bis 3 g /100 mL EPA, 0,5 g / 100 mL bis 2,5 g /100 mL EPA, 0,5 g / 100 mL bis 2 g /100 mL EPA, 0,5 g / 100 mL bis 1,5 g / 100 mL EPA oder 0,5 g / 100 mL bis 1,0 g / 100 mL EPA umfassen.

Vorzugsweise umfasst die Zusammensetzung 1,0 g / 100 mL bis 7,0 g /100 mL EPA. Ganz besonders bevorzugt ist eine Zusammensetzung umfassend 1,0 g / 100 mL bis 4,0 g / 100 mL EPA.

Die Zusammensetzung kann 0,5 g / 100 mL bis 10,0 g / 100 mL DHA umfassen. Die Zusammensetzung kann 1 g / 100 mL bis 10,0 g / 100 mL DHA, 1,5 g / 100 mL bis 10,0 g / 100 mL DHA, 2g / 100 mL bis 10,0 g / 100 mL DHA, 2,5 g / 100 mL bis 10,0 g / 100 mL DHA, 3 g / 100 mL bis 10,0 g / 100 mL DHA, 3,5 g / 100 mL bis 10,0 g / 100 mL DHA, 4 g / 100 mL bis 10,0 g / 100 mL DHA, 4,5 g / 100 mL bis 10,0 g / 100 mL DHA, 5 g / 100 mL bis 10,0 g / 100 mL DHA, 5,5 g / 100 mL bis 10,0 g / 100 mL DHA, 6 g / 100 mL bis 10,0 g / 100 mL DHA, 6,5 g / 100 mL bis 10,0 g / 100 mL DHA, 7 g / 100 mL bis 10,0 g / 100 mL DHA, 7,5 g / 100 mL bis 10,0 g / 100 mL DHA, 8 g / 100 mL bis 10,0 g / 100 mL DHA, 8,5 g / 100 mL bis 10,0 g /100 mL DHA, 9 g / 100 mL bis 10,0 g /100 mL DHA oder 9,5 g / 100 mL bis 10,0 g /100 mL DHA umfassen.

Die Zusammensetzung kann 0,5 g / 100 mL bis 9,5 g / 100 mL DHA, 0,5 g / 100 mL bis 9 g / 100 mL DHA, 0,5 g / 100 mL bis 8,5 g / 100 mL DHA, 0,5 g / 100 mL bis 8 g /100 mL DHA, 0,5 g / 100 mL bis 7,5 g / 100 mL DHA, 0,5 g / 100 mL bis 7 g / 100 mL DHA, 0,5 g / 100 mL bis 6,5 g / 100 mL DHA, 0,5 g / 100 mL bis 6 g / 100 mL DHA, 0,5 g / 100 mL bis 5,5 g / 100 mL DHA, 0,5 g / 100 mL bis 5 g / 100 mL DHA, 0,5 g / 100 mL bis 4,5 g / 100 mL DHA, 0,5 g / 100 mL bis 4 g / 100 mL DHA, 0,5 g / 100 mL bis 3,5 g / 100 mL DHA, 0,5 g / 100 mL bis 3 g /100 mL DHA, 0,5 g / 100 mL bis 2,5 g / 100 mL DHA, 0,5 g / 100 mL bis 2 g / 100 mL DHA, 0,5 g / 100 mL bis 1,5 g / 100 mL DHA oder 0,5 g / 100 mL bis 1,0 g / 100 mL DHA umfassen.

Vorzugsweise umfasst die Zusammensetzung 1,0 g/ 100 mL bis 7,0 g/ 100 mL DHA. Ganz besonders bevorzugt ist eine Zusammensetzung enthaltend 1,0 g / 100 mL bis 4,0 g / 100 mL DHA.

Die erfindungsgemäße Zusammensetzung kann 5 g / 100 mL bis 50 g / 100 mL hochaufgereinigtes Fischöl umfassen. Die Zusammensetzung kann 10 g / 100 mL bis 50 g / 100 mL hochaufgereinigtes Fischöl, 15 g / 100 mL bis 50 g / 100 mL hochaufgereinigtes Fischöl, 20 g / 100 mL bis 50 g / 100 mL hochaufgereinigtes Fischöl, 25 g / 100 mL bis 50 g / 100 mL hochaufgereinigtes Fischöl, 30 g / 100 mL bis 50 g /100 mL hochaufgereinigtes Fischöl, 35 g / 100 mL bis 50 g /100 mL hochaufgereinigtes Fischöl, 40 g / 100 mL bis 50 g /100 mL hochaufgereinigtes Fischöl, oder 45 g / 100 mL bis 50 g / 100 mL hochaufgereinigtes Fischöl umfassen. Die Zusammensetzung kann 5 g / 100 mL bis 45 g /100 mL hochaufgereinigtes Fischöl, 5 g / 100 mL bis 40 g / 100 mL hochaufgereinigtes Fischöl, 5 g / 100 mL bis 35 g / 100 mL hochaufgereinigtes Fischöl, 5 g / 100 mL bis 30 g /100 mL hochaufgereinigtes Fischöl, 5 g / 100 mL bis 25 g /100 mL hochaufgereinigtes Fischöl, 5 g / 100 mL bis 20 g /100 mL hochaufgereinigtes Fischöl, 5 g / 100 mL bis 15 g /100 mL hochaufgereinigtes Fischöl, oder 5 g / 100 mL bis 10 g /100 mL hochaufgereinigtes Fischöl umfassen. Bevorzugt ist eine Zusammensetzung, die 10 g / 100 mL bis 40 g /100 mL hochaufgereinigtes Fischöl, beispielsweise 15 g / 100 mL bis 35 g /100 mL hochaufgereinigtes Fischöl umfasst.

Die Zusammensetzung kann EPA, DHA oder eine Kombination davon umfassen.

Bevorzugt ist die Verwendung von Omegaven® (Fresenius Kabi) als erfindungsgemäße Zusammensetzung.

Die Zusammensetzung kann neben Omeg-3-Fettsäuren auch weitere Zusatzstoffe enthalten. Die erfindungsgemäße Zusammensetzung enthält in bevorzugter Weise weitere Zusatzstoffe ausgewählt aus mittelkettigen Fettsäuren (MCT) oder Eisen, oder einer Kombination davon. Die Erfinder haben überraschend herausgefunden, dass durch die Zugabe von MCT und / oder Eisen zu der Zusammensetzung ein synergistischer Effekt mit den in der erfindungsgemäßen Zusammensetzung enthaltenen Omega-3-Fettsäuren auftritt, der die Wirksamkeit der Zusammensetzung noch verbessert.

Die erfindungsgemäße Zusammensetzung kann 5 g/ 100 mL bis 50 g/ 100 mL MCT umfassen.

Die Zusammensetzung kann 10 g / 100 mL - 50 g / 100 mL MCT, 15 g / 100 mL - 50 g / 100 mL MCT, 20 g / 100 mL - 50 g / 100 mL MCT, 25 g / 100 mL - 50 g / 100 mL MCT, 30 g / 100 mL - 50 g /100 mL MCT, 35 g / 100 mL - 50 g /100 mL MCT, 40 g / 100 mL - 50 g /100 mL MCT, oder 45 g / 100 mL - 50 g /100 mL MCT umfassen. Die Zusammensetzung kann 5 g / 100 mL - 45 g /100 mL MCT, 5 g / 100 mL - 40 g /100 mL MCT, 5 g / 100 mL - 35 g /100 mL MCT, 5 g / 100 mL - 30 g /100 mL MCT, 5 g / 100 mL - 25 g /100 mL MCT, 5 g / 100 mL - 20 g /100 mL MCT, 5 g / 100 mL - 15 g /100 mL MCT, oder 5 g / 100 mL - 10 g /100 mL MCT umfassen. Bevorzugt ist eine Zusammensetzung, die 10 g / 100 mL - 40 g /100 mL MCT, beispielsweise 15 g / 100 mL - 35 g /100 mL MCT umfasst.

Die erfindungsgemäße Zusammensetzung kann 0,1 mg /100 mL - 0,5 mg / 100 mL Eisen umfassen. Die Zusammensetzung kann 0,15 mg / 100 mL - 0,5 mg / 100 mL Eisen, 0,2 mg /100 mL - 0,5 mg / 100 mL Eisen, 0,25 mg /100 mL - 0,5 mg / 100 mL Eisen, 0,3 mg / 100 mL - 0,5 mg / 100 mL Eisen, 0,35 mg /100 mL - 0,5 mg / 100 mL Eisen, 0,4 mg /100 mL - 0,5 mg / 100 mL Eisen oder 0,45 mg /100 mL - 0,5 mg / 100 mL Eisen umfassen. Die Zusammensetzung kann 0,1 mg /100 mL - 0,45 mg / 100 mL Eisen, 0,1 mg /100 mL - 0,4 mg / 100 mL Eisen, 0,1 mg /100 mL - 0,35 mg / 100 mL Eisen, 0,1 mg /100 mL - 0,3mg / 100 mL Eisen, 0,1 mg /100 mL - 0,25 mg / 100 mL Eisen, 0,1 mg /100 mL - 0,2 mg / 100 mL Eisen, 0,1 mg /100 mL - 0,15 mg / 100 mL Eisen umfassen. Bevorzugt ist eine Zusammensetzung, die 0,15 mg /100 mL - 0,45 mg / 100 mL Eisen umfasst, beispielsweise 0,2 mg /100 mL - 0,4 mg / 100 mL Eisen.

Die Zusammensetzung wird vor dem Beginn eines Zyklus einer Chemotherapie oder Strahlentherapie verabreicht. Die Zusammensetzung kann 96 Stunden bis 24 Stunden vor dem Beginn eines Zyklus verabreicht werden, in bevorzugter Weise 72 bis 24 Stunden vor dem Beginn eines Zyklus Ganz besonders bevorzugt wird die Zusammensetzung 48 bis 24 Stunden vor dem Beginn eines Zyklus der Chemotherapie oder der Strahlentherapie verabreicht wird.

Zusätzlich zu der vorstehend beschriebenen Verabreichung vor dem Beginn eines Zyklus einer Chemotherapie oder Strahlentherapie kann die Zusammensetzung auch noch zwischen 24 und 1 Stunde vor dem Beginn des Zyklus verabreicht werden. Hierbei ist eine Verabreichung zwischen 10 und 2 Stunden vor dem Beginn des Zyklus vorteilhaft, ganz besonders bevorzugt ist eine Verabreichung 3 Stunden vor dem Beginn eines Zyklus.

Die erfindungsgemäße Zusammensetzung kann grundsätzlich einmal oder mehrmals vor dem Beginn eines Zyklus einer Chemotherapie oder Strahlentherapie verabreicht werden. Bei mehrmaliger Verabreichung kann die Zusammensetzung zum Beispiel zweimal, dreimal, viermal, oder fünfmal verabreicht werden. Die Zusammensetzung kann konsekutiv verabreicht werden.

Üblicherweise umfasst eine Chemotherapie oder Strahlentherapie mehrere Zyklen. Hier kann die Zusammensetzung kann vor dem Beginn eines Zyklus oder vor mehreren Zyklen, jeweils vor dem Beginn des Zyklus, verabreicht werden. Die Zusammensetzung kann vor jedem der Zyklen verabreicht werden.

Die Zusammensetzung kann konsekutiv an mehreren aufeinanderfolgenden Tagen verabreicht werden, wobei die Zusammensetzung bevorzugt an drei aufeinanderfolgenden Tagen verabreicht wird.

Die Zusammensetzung kann parenteral, vorzugsweise intravenös verabreicht werden.

Mit der Zusammensetzung können 5 mg bis 250 mg, zum Beispiel 10 mg bis 250 mg EPA pro Kilogramm Körpergewicht und pro Tag verabreicht werden. Es können vorzugsweise 25 mg bis 250 mg EPA, 50 mg bis 250 mg EPA, 75 mg bis 250 mg EPA, 100 mg bis 250 mg EPA, 125 mg bis 250 mg EPA, 150 mg bis 250 mg EPA, 175 mg bis 250 mg EPA, 200 mg bis 250 mg EPA, 225 mg bis 250 mg EPA pro Kilogramm Körpergewicht und pro Tag verabreicht werden. Es können 5 mg bis 10 mg EPA, 5 mg bis 25 mg EPA, 5 mg bis 50 mg EPA, 5 mg bis 75 mg EPA, 5 mg bis 100 mg EPA, 5 mg bis 125 mg EPA, 5 mg bis 150 mg EPA, 5 mg bis 175 mg EPA, 5 mg bis 200 mg EPA oder 5 mg bis 225 mg EPA pro Kilogramm Körpergewicht und pro Tag verabreicht werden.

Bevorzugt ist es, mit der Zusammensetzung 5 mg bis 100 mg EPA, beispielsweise 15 mg bis 85 mg EPA, 20 mg bis 80 mg EPA, 25 mg bis 75 mg EPA, 30 mg bis 70 mg EPA, 35 mg bis 65 mg EPA, oder 40 mg bis 60 mg EPA pro Kilogramm Körpergewicht und pro Tag zu verabreichen. Ganz besonders bevorzugt ist eine Verabreichung von 45 mg bis 55 mg EPA, 45 mg EPA, 46 mg EPA, 47 mg EPA, 48 mg EPA, 49 mg EPA, 50 mg EPA, 51 mg EPA, 52 mg EPA, 53 mg EPA, 54 mg EPA, oder 55 mg EPA pro Kilogramm Körpergewicht und pro Tag.

Mit der Zusammensetzung können 5 mg bis 250 mg, zum Beispiel 10 mg bis 250 mg DHA pro Kilogramm Körpergewicht und pro Tag verabreicht werden. Es können vorzugsweise 25 mg bis 250 mg DHA, 50 mg bis 250 mg DHA, 75 mg bis 250 mg DHA, 100 mg bis 250 mg DHA, 125 mg bis 250 mg DHA, 150 mg bis 250 mg DHA, 175 mg bis 250 mg DHA, 200 mg bis 250 mg DHA, 225 mg bis 250 mg DHA pro Kilogramm Körpergewicht und pro Tag verabreicht werden. Es können 5 mg bis 10 mg DHA, 5 mg bis 25 mg DHA, 5 mg bis 50 mg DHA, 5 mg bis 75 mg DHA, 5 mg bis 100 mg DHA, 5 mg bis 125 mg DHA, 5 mg bis 150 mg DHA, 5 mg bis 175 mg DHA, 5 mg bis 200 mg DHA oder 5 mg bis 225 mg DHA pro Kilogramm Körpergewicht und pro Tag verabreicht werden.

Bevorzugt ist es, mit der Zusammensetzung 5 mg bis 100 mg DHA, beispielsweise 15 mg bis 85 mg DHA, 20 mg bis 80 mg DHA, 25 mg bis 75 mg DHA, 30 mg bis 70 mg DHA, 35 mg bis 65 mg DHA, oder 40 mg bis 60 mg DHA pro Kilogramm Körpergewicht und pro Tag zu verabreichen. Ganz besonders bevorzugt ist eine Verabreichung von 45 mg bis 55 mg DHA, 45 mg DHA, 46 mg DHA, 47 mg DHA, 48 mg DHA, 49 mg DHA, 50 mg DHA, 51 mg DHA, 52 mg DHA, 53 mg DHA, 54 mg DHA, oder 55 mg DHA pro Kilogramm Körpergewicht und pro Tag.

Mit der Zusammensetzung können 0,025 g bis 1,25 g Fischöl pro Kilogramm Körpergewicht und pro Tag verabreicht werden. Es können beispielsweise 0,05 g bis 1,25 g Fischöl, 0,075 g bis 1,25 g Fischöl, 0,1 g bis 1,25 g Fischöl, 0,2 g bis 1,25 g Fischöl, 0,3 g bis 1,25 g Fischöl, 0,4 g bis 1,25 g Fischöl, 0, 5 g bis 1,25 g Fischöl, 0,6 g bis 1,25 g Fischöl, 0,7 g bis 1,25 g Fischöl, 0,8 g bis 1,25 g Fischöl, 0,9 g bis 1,25 g Fischöl oder 1,0 g bis 1,25 g Fischöl pro Kilogramm Körpergewicht und pro Tag verabreicht werden. Es können 1,0 g bis 0,025 g Fischöl, 1,0 g bis 0,025 g Fischöl, 0,9 g bis 0,025 g Fischöl, 0,8 g bis 0,025 g Fischöl, 0,7 g bis 0,025 g Fischöl, 0,6 g bis 0,025 g Fischöl, 0,5 g bis 0,025 g Fischöl, 0,4 g bis 0,025 g Fischöl, 0,3 g bis 0,025 g Fischöl, 0,3 g bis 0,025 g Fischöl, 0,2 g bis 0,025 g Fischöl, 0,1 g bis 0,025 g Fischöl, 0,075 g bis 0,025 g Fischöl oder 0,05 g bis 0,025 g Fischöl pro Kilogramm Körpergewicht und pro Tag verabreicht werden.

Bevorzugt ist die Verabreichung von 0,1 g bis 0,7 g Fischöl, 0,15 g bis 0,65 g Fischöl, 0,2 g bis 0,6 g Fischöl, 0,25 g bis 0,55 g Fischöl pro Kilogramm Körpergewicht und pro Tag. Ganz besonders bevorzugt ist die Verabreichung von 0,3 g bis 0,5 g Fischöl pro Kilogramm Körpergewicht und pro Tag. Und ebenfalls bevorzugt ist die Verabreichung von 0,2 g Fischöl pro Kilogramm Körpergewicht und pro Tag. Das Fischöl kann hochaufgereinigt sein.

Am meisten bevorzugt ist die Verwendung von Omegaven® (Fresenius Kabi) als erfindungsgemäße Zusammensetzung. Omegaven® kann in einer Dosierung von 0,25 mL bis 12,5 mL pro kg Körpergewicht und Tag verabreicht werden. Zum Beispiel können 0,5 mL bis 12,5 mL, 1,0 mL bis 12,5 mL, 1,5 mL bis 12,5 mL, 2,0 mL bis 12,5 mL, 2,5 mL bis 12,5 mL, 3,0 mL bis 12,5 mL, 3,5 mL bis 12,5 mL, 4,0 mL bis 12,5 mL, 4,5 mL bis 12,5 mL, 5,0 mL bis 12,5 mL, 5,5 mL bis 12,5 mL, 6,0 mL bis 12,5 mL, 7,0 mL bis 12,5, 8,0 mL bis 12,5 mL, 9,0 mL bis 12,5 mL, 10,0 mL bis 12,5 mL mL, 11,0 mL bis 12,5 mL, 11,5 mL bis 12,5 mL oder 12,0 mL bis 12,5 mL Omegaven® pro kg Körpergewicht und Tag verabreicht werden. Es können 12,0 mL bis 0,25 mL, 11,5 mL bis 0,25 mL, 11,0 mL bis 0,25 mL, 10,0 mL bis 0,25 mL, 9,0 mL bis 0,25 mL, 8,0 mL bis 0,25 mL, 7,0 mL bis 0,25 mL, 6,0 mL bis 0,25 mL, 5,5 mL bis 0,25 mL, 4,0 mL bis 0,25 mL, 3,5 mL bis 0,25 mL, 3,0 mL bis 0,25 mL, 2,5 mL bis 0,25 mL, 2,0 mL bis 0,25 mL, 1,5 mL bis 0,25 mL, 1,0 mL bis 0,25 mL, 0,75 mL bis 0,25 mL, oder 0,5 mL bis 0,25 mL Omegaven® pro Kilogramm Körpergewicht und pro Tag verabreicht werden.

Bevorzugt ist eine Verabreichung von 1,0 mL bis 7,0 mL, 1,5 mL bis 6,5 mL, 2,0 mL bis 6,0 mL oder 2,5 mL bis 5,5 mL Omegaven® pro Kilogramm Körpergewicht und pro Tag. Ganz besonders bevorzugt ist eine Verabreichung von 3,0 mL bis 5,0 mL Omegaven® pro Kilogramm Körpergewicht und pro Tag. Ebenfalls bevorzugt ist die Verabreichung von 2,0 mL Omegaven® pro Kilogramm Körpergewicht und pro Tag.

Mit der Zusammensetzung können 0,07 g bis 0,7 g MCT pro Kilogramm Körpergewicht und pro Tag verabreicht werden. Es können 0,1 g bis 0,7 g MCT, 0,2 g bis 0,7 g MCT, 0,25 g bis 0,7 g MCT, 0,3 g bis 0,7 g MCT, 0,35 g bis 0,7 g MCT, 0,4 g bis 0,7 g MCT, 0,45 g bis 0,7 g MCT, 0,5 g bis 0,7 g MCT, 0,55 g bis 0,7 g MCT, 0,6 g bis 0,7 g MCT oder 0,65 g bis 0,7 g MCT pro Kilogramm Körpergewicht und pro Tag verabreicht werden. Mit der Zusammensetzung können 0,65 g bis 0,07 g MCT, 0,6 g bis 0,07 g MCT, 0,55 g bis 0,07 g MCT, 0,5 g bis 0,07 g MCT, 0,45 g bis 0,07 g MCT, 0,4 g bis 0,07 g MCT, 0,35 g bis 0,07 g MCT, 0,3 g bis 0,07 g MCT, 0,25 g bis 0,07 g MCT, 0,2 g bis 0,07 g MCT, 0,15 g bis 0,07 g MCT oder 0,1 g bis 0,07 g MCT pro Kilogramm Körpergewicht und pro Tag verabreicht werden.

Bevorzugt ist die Verabreichung von 0,1 g bis 0,5 g MCT, 0,2 g bis 0,6 g MCT, 0,3 g bis 0,5 g MCT oder 0,35 g bis 0,45 g MCT pro Kilogramm Körpergewicht und pro Tag.

Mit der Zusammensetzung können 1 mg bis 11 mg Eisen pro Tag verabreicht werden. Es können 1 mg -10 mg Eisen, 1 mg -9 mg Eisen, 1 mg -8 mg Eisen, 1 mg -7 mg Eisen, 1 mg -6 mg Eisen, 1 mg - 5 mg Eisen, 1 mg - 4 mg Eisen, 1 mg - 3 mg Eisen, 1 mg - 2 mg Eisen pro Tag verabreicht werden. Es können 2 mg - 11 mg Eisen, 3 mg - 11 mg Eisen, 4 mg - 11 mg Eisen, 5 mg - 11 mg Eisen, 6 mg - 11 mg Eisen, 7 mg - 11 mg Eisen, 8 mg - 11 mg Eisen, 9 mg - 11 mg Eisen, oder 10 mg - 11 mg Eisen pro Tag verabreicht werden. Bevorzugt ist eine Verabreichung von 3 mg -9 mg Eisen pro Tag.

Bereitgestellt wird eine Zusammensetzung mit einem Volumen von 100 mL, umfassend Omega-3-Fettsäuren. Die Zusammensetzung kann eine oder mehrere verschiedene Omega-3-Fettsäuren umfassen. Bevorzugt umfasst die Zusammensetzung Omega-3-Fettsäuren ausgewählt aus der Gruppe bestehend aus EPA, DHA, langkettigen und überlangkettigen Omega-3-Fettsäuren oder einer Kombination davon. Besonders bevorzugt ist eine Zusammensetzung mit einem Volumen von 100 mL, die 0,5 g bis 10 g EPA und / oder 0,5 g bis 10 g DHA umfasst. Die Zusammensetzung kann Fischöl umfassen, beispielsweise 5 g / 100 mL bis 50 g /100 mL. Die bereitgestellte Zusammensetzung kann ferner MCTs und / oder Eisen umfassen. Die Zusammensetzung kann 5 g / 100 mL bis 50 g / 100 mL MCTs umfassen und / oder 0,1 mg /100 mL - 0,5 mg / 100 mL Eisen.

Die bereitgestellte erfindungsgemäße Zusammensetzung mit einem Volumen von 100 mL kann 0,5 g bis 10,0 g EPA umfassen. Die Zusammensetzung kann 1 g bis 10,0 g EPA, 1,5 g bis 10,0 g EPA, 2 g bis 10,0 g EPA, 2,5 g bis 10,0 g EPA, 3 g bis 10,0 g EPA, 3,5 g bis 10,0 g EPA, 4 g bis 10,0 g EPA, 4,5 g bis 10,0 g EPA, 5 g bis 10,0 g EPA, 5,5 g bis 10,0 g EPA, 6 g bis 10,0 g EPA, 6,5 g bis 10,0 g EPA, 7 g bis 10,0 g EPA, 7,5 g bis 10,0 g EPA, 8 g bis 10,0 g EPA, 8,5 g bis 10,0 g EPA, 9 g bis 10,0 g EPA oder 9,5 g bis 10,0 g EPA umfassen.

Die Zusammensetzung kann 0,5 g bis 9,5 g EPA, 0,5 g bis 9 g EPA, 0,5 g bis 8,5 g EPA, 0,5 g bis 8 g EPA, 0,5 g bis 7,5 g EPA, 0,5 g bis 7 g EPA, 0,5 g bis 6,5 g EPA, 0,5 g bis 6 g EPA, 0,5 g bis 5,5 g EPA, 0,5 g bis 5 g EPA, 0,5 g bis 4,5 g EPA, 0,5 g bis 4 g EPA, 0,5 g bis 3,5 g EPA, 0,5 g bis 3 g EPA, 0,5 g bis 2,5 g EPA, 0,5 g bis 2 g EPA, 0,5 g bis 1,5 g EPA oder 0,5 g bis 1,0 g EPA umfassen.

Vorzugsweise umfasst die Zusammensetzung 1,0 g bis 7,0 g EPA. Ganz besonders bevorzugt ist eine Zusammensetzung umfassend 1,0 g bis 4,0 g EPA.

Die bereitgestellte erfindungsgemäße Zusammensetzung mit einem Volumen von 100 mL kann 0,5 g bis 10,0 g DHA umfassen. Die Zusammensetzung kann 1 g bis 10,0 g DHA, 1,5 g bis 10,0 g DHA, 2 g bis 10,0 g DHA, 2,5 g bis 10,0 g DHA, 3 g bis 10,0 g DHA, 3,5 g bis 10,0 g DHA, 4 g bis 10,0 g DHA, 4,5 g bis 10,0 g DHA, 5 g bis 10,0 g DHA, 5,5 g bis 10,0 g DHA, 6 g bis 10,0 g DHA, 6,5 g bis 10,0 g DHA, 7 g bis 10,0 g DHA, 7,5 g bis 10,0 g DHA, 8 g bis 10,0 g DHA, 8,5 g bis 10,0 g DHA, 9 g bis 10,0 g DHA oder 9,5 g bis 10,0 g DHA umfassen.

Die bereitgestellte erfindungsgemäße Zusammensetzung mit einem Volumen von 100 mL kann 0,5 g bis 9,5 g DHA, 0,5 g bis 9 g DHA, 0,5 g bis 8,5 g DHA, 0,5 g bis 8 g DHA, 0,5 g bis 7,5 g DHA, 0,5 g bis 7 g DHA, 0,5 g bis 6,5 g DHA, 0,5 g bis 6 g DHA, 0,5 g bis 5,5 g DHA, 0,5 g bis 5 g DHA, 0,5 g bis 4,5 g DHA, 0,5 g bis 4 g DHA, 0,5 g bis 3,5 g DHA, 0,5 g bis 3 g DHA, 0,5 g bis 2,5 g DHA, 0,5 g bis 2 g DHA, 0,5 g bis 1,5 g DHA oder 0,5 g bis 1,0 g DHA umfassen.

Vorzugsweise umfasst die Zusammensetzung 1,0 g bis 7,0 g DHA. Ganz besonders bevorzugt ist eine Zusammensetzung umfassend 1,0 g bis 4,0 g DHA.

Die bereitgestellte erfindungsgemäße Zusammensetzung mit einem Volumen von 100 mL kann 5 g - 50 g hochaufgereinigtes Fischöl umfassen. Die Zusammensetzung kann 10 g - 50 g hochaufgereinigtes Fischöl, 15 g - 50 g hochaufgereinigtes Fischöl, 20 g - 50 g hochaufgereinigtes Fischöl, 25 g - 50 g hochaufgereinigtes Fischöl, 30 g - 50 g hochaufgereinigtes Fischöl, 35 g - 50 g hochaufgereinigtes Fischöl, 40 g - 50 g hochaufgereinigtes Fischöl, oder 45 g - 50 g hochaufgereinigtes Fischöl umfassen. Die Zusammensetzung kann 5 g - 45 g hochaufgereinigtes Fischöl, 5 g - 40 g hochaufgereinigtes Fischöl, 5 g - 35 g hochaufgereinigtes Fischöl, 5 g - 30 g hochaufgereinigtes Fischöl, 5 g - 25 g hochaufgereinigtes Fischöl, 5 g - 20 g hochaufgereinigtes Fischöl, 5 g - 15 g hochaufgereinigtes Fischöl, oder 5 g - 10 g hochaufgereinigtes Fischöl umfassen Bevorzugt ist eine Zusammensetzung, die 10 g - 40 g hochaufgereinigtes Fischöl, beispielsweise 15 g - 35 g hochaufgereinigtes Fischöl umfasst.

Die Zusammensetzung kann EPA, DHA oder eine Kombination davon umfassen.

Die bereitgestellte erfindungsgemäße Zusammensetzung mit einem Volumen von 100 mL kann 5 g bis 50 g MCT umfassen. Die Zusammensetzung kann 10 g bis 50 g MCT, 15 g bis 50 g MCT, 20 g bis 50 g MCT, 25 g bis 50 g MCT, 30 g bis 50 g MCT, 35 g bis 50 g MCT, 40 g bis 50 g MCT, oder 45 g bis 50 g MCT umfassen. Die Zusammensetzung kann 5 g bis 10 g MCT, 5 g bis 15 g MCT, 5 g bis 20 g MCT, 5 g bis 25 g MCT, 5 g bis 30 g MCT, 5 g bis 35 g MCT, 5 g bis 40 g MCT, oder 5 g bis 45 g MCT umfassen. Bevorzugt ist eine Zusammensetzung, die 10 g bis 40 g MCT umfasst, beispielsweise 15 g bis 35 g.

Die bereitgestellte erfindungsgemäße Zusammensetzung mit einem Volumen von 100 mL kann 0,1 mg - 0,5 mg Eisen umfassen. Die Zusammensetzung kann 0,15 mg - 0,5 mg Eisen, 0,2 mg - 0,5 mg Eisen, 0,25 mg - 0,5 mg Eisen, 0,3 mg - 0,5 mg Eisen, 0,35 mg - 0,5 mg Eisen, 0,4 mg - 0,5 mg Eisen oder 0,45 mg - 0,5 mg Eisen umfassen.

Die Zusammensetzung kann 0,1 mg - 0,45 mg Eisen, 0,1 mg - 0,4 mg Eisen, 0,1 mg - 0,35 mg Eisen, 0,1 mg - 0,3mg Eisen, 0,1 mg - 0,25 mg Eisen, 0,1 mg - 0,2 mg Eisen, 0,1 mg - 0,15 mg Eisen umfassen. Bevorzugt ist eine Zusammensetzung, die 0,15 mg - 0,45 mg Eisen umfasst, beispielsweise 0,2 mg - 0,4 mg Eisen.

Offenbart wird eine Zusammensetzung mit einem Volumen von 50 mL, die sich von der vorstehend offenbarten Lösung mit einem Volumen von 100 mL dadurch unterscheidet, dass sie jeweils nur die Hälfte der Menge eines Inhaltsstoffs umfasst, die für die Zusammensetzung mit dem Volumen von 100 mL offenbart ist.

Bereitgestellt wird ein Verfahren zur Verabreichung der erfindungsgemäßen Zusammensetzung.

Bereitgestellt wird ein Verfahren zur Verabreichung einer Zusammensetzung umfassend Omega-3-Fettsäuren ausgewählt aus Eicosapentaensäure (EPA) und Docosahexaensäure (DHA), oder einer Kombination davon, an einen an Krebs erkrankten Patienten, zur Verbesserung der Wirksamkeit einer Chemotherapie oder einer Strahlentherapie und / oder zur Vorbeugung oder Reduktion von durch die Chemotherapie oder die Strahlentherapie hervorgerufenen Nebenwirkungen, das Verfahren umfassend die folgenden Schritte: (a) die Verabreichung der Zusammensetzung vor Beginn eines Zyklus der Chemotherapie oder der Strahlentherapie an den Patienten; (b) Behandlung des Patienten mit mindestens einem Zyklus einer Chemotherapie oder Strahlentherapie.

Bereitgestellt wird das Verfahren zur Verabreichung einer Zusammensetzung umfassend Omega-3-Fettsäuren ausgewählt aus Eicosapentaensäure (EPA) und Docosahexaensäure (DHA), oder einer Kombination davon, an einen an Krebs erkrankten Patienten, zur Verbesserung der Wirksamkeit einer Chemotherapie oder einer Strahlentherapie und / oder zur Vorbeugung oder Reduktion von durch die Chemotherapie oder die Strahlentherapie hervorgerufenen Nebenwirkungen, das Verfahren umfassend die folgenden Schritte: (a) die Verabreichung der Zusammensetzung vor Beginn eines Zyklus der Chemotherapie oder der Strahlentherapie, wobei die Zusammensetzung 96 Stunden bis 24 Stunden vor dem Beginn eines Zyklus der Chemotherapie oder der Strahlentherapie verabreicht wird, vorzugsweise wobei die Zusammensetzung 72 bis 24 Stunden vor dem Beginn eines Zyklus der Chemotherapie oder der Strahlentherapie verabreicht wird, ganz besonders bevorzugt wobei die Zusammensetzung 48 bis 24 Stunden vor dem Beginn eines Zyklus der Chemotherapie oder der Strahlentherapie verabreicht wird; (b) Behandlung des Patienten mit mindestens einem Zyklus einer Chemotherapie oder Strahlentherapie.

Die Verabreichung unter Schritt (a) kann einmal oder mehrmals erfolgen, zum Beispiel zweimal, dreimal, viermal oder fünfmal. Der Patient kann unter Schritt (b) beispielsweise mit 1, 2, 3,4 5, 6, 7, 8, 9, 10, 11, 12, 13, 14, oder 15 Zyklen einer Chemotherapie oder Strahlentherapie behandelt werden.

Das Verfahren kann ferner umfassen den Schritt: (a)' Zusätzlich Verabreichung der Zusammensetzung auch noch zwischen 24 und 1 Stunde vor dem Beginn des Zyklus der Chemotherapie oder Strahlentherapie verabreicht wird, vorzugsweise wobei die Zusammensetzung 3 Stunden vor dem Beginn eines Zyklus der Chemotherapie oder der Strahlentherapie verabreicht wird. Die Verabreichung unter Schritt (a)' kann einmal oder mehrmals erfolgen, zum Beispiel zweimal, dreimal, viermal oder fünfmal.

Das Verfahren kann so durchgeführt werden, dass 5 mg bis 250 mg pro Kilogramm Körpergewicht und pro Tag EPA, vorzugsweise wobei 20 mg bis 80 mg pro Kilogramm Körpergewicht und pro Tag EPA, am meisten bevorzugt wobei 40 mg bis 60 mg pro Kilogramm Körpergewicht und pro Tag EPA verabreicht werden und / oder dass 5 mg bis 250 mg pro Kilogramm Körpergewicht und pro Tag DHA, vorzugsweise wobei 20 mg bis 80 mg pro Kilogramm Körpergewicht und pro Tag DHA, am meisten bevorzugt wobei 40 mg bis 60 mg pro Kilogramm Körpergewicht und pro Tag DHA verabreicht werden.

Die Schritte (a) und / oder (a)' des Verfahrens können wiederholt werden, wobei die vorzugsweise Zusammensetzung konsekutiv an mehreren aufeinanderfolgenden Tagen verabreicht wird, bevorzugt wobei die Zusammensetzung an drei aufeinanderfolgenden Tagen verabreicht wird.

Die Verabreichung der Zusammensetzung kann parenteral, vorzugsweise intravenös erfolgen.

Das Verfahren kann zusätzlich umfassen den Schritt: (c) Zusätzliche Verabreichung der Zusammensetzung während oder nach einem Zyklus der Chemotherapie oder Strahlentherapie.

Das Verfahren kann zur Vorbeugung oder Reduktion von durch die Chemotherapie oder die Strahlentherapie hervorgerufenen Nebenwirkungen eingesetzt werden, wobei die Nebenwirkungen bevorzugt ausgewählt sind aus der Gruppe bestehend aus gastrointestinale Nebenwirkungen, hämatologische Nebenwirkungen, Reduktion des Lebergewichts, neurotoxische Nebenwirkungen, das Herz betreffende Nebenwirkungen, inflammatorische Nebenwirkungen, Gewichtsverlust, eingeschränkte Funktion der Immunabwehr, Reduktion von Entzündungen oder einer Kombination davon.

Das Verfahren kann zur Behandlung von Krebserkrankungen eingesetzt werden, wobei die Krebserkrankung ausgewählt ist aus der Gruppe bestehend aus der Gruppe bestehend aus soliden Tumoren und nicht-soliden Tumoren, vorzugsweise wobei die soliden Tumoren ausgewählt sind aus der Gruppe bestehend aus kolorektales Karzinom, Mammakarzinom, Pankreaskarzinom, Leberkarzinom, Lungenkarzinom, und Magenkarzinom.

Das Verfahren kann bei Patienten eingesetzt werden, die eine Chemotherapie erhalten, bevorzugt wobei die Chemotherapie ein Chemotherapeutikum umfasst, das ausgewählt ist aus der Gruppe bestehend aus 5-Fluorouracil, Gemzitabin, Doxorubicin, Paclitaxel, Mitomycin, Cyclophosphamid, Epirubicin, Arabinosylcytosin, Tamoxifen, Irinotecan, Oxaliplatin, Folinsäure, Cisplatin, Taxane, Vinca Alkaloide, Epipodophyllotoxine, synthetische Alkaloide, Cytarabine, Nitrosurea, Dacarbazine, Fludarabine, Ifosfamid, Mytomicin C, Tamoxifen oder einer Kombination davon.

Besonders bevorzugt ist das Verfahren, wobei die Chemotherapie 5-Fluorouracil umfasst, ganz besonders bevorzugt wobei die Chemotherapie FOLFOX oder FOLFIRI ist.

Das Verfahren kann bei Patienten eingesetzt werden, die eine Strahlentherapie erhalten, bevorzugt wobei die Strahlentherapie ausgewählt ist aus der Gruppe bestehend aus Teletherapie und Brachytherapie.

Die mittels des Verfahrens verabreichte Zusammensetzung kann weitere bevorzugte Zusatzstoffe ausgewählt aus der Gruppe bestehend aus mittelkettige Fettsäuren und Eisen, oder einer Kombination davon enthalten.

Die mittels des Verfahrens verabreichte Zusammensetzung kann 0,5 g / 100 mL bis 10,0 g /100 mL EPA und /oder 0,5 g/ 100 mL bis 10,0 g /100 mL DHA enthalten, vorzugsweise 0,7 g / 100 mL bis 3,5 g /100 mL EPA und /oder 0,7 g / 100 mL bis 3,5 g /100 mL DHA. Ganz besonders bevorzugt enthält die Zusammensetzung 1 g / 100 mL bis 3,1 g / 100 mL EPA und / oder DHA.

Die vorstehend genannten Werte und Bereiche sind einzeln und in Kombination umfasst. Jeder Wert eines Bereichs ist einzeln umfasst, insbesondere alle ganzzahligen Zwischenwerte.

Beispiele

**Beispiel 1:** Herstellung von Zusammensetzung umfassend Eicosapentaensäure (EPA) und Docosahexaensäure (DHA).

**a)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |

**b)** Eine Zusammensetzung umfassend die folgenden Bestandteile (Angaben bezogen auf 100 mL sofern nicht anders angegeben) wurde hergestellt:

| | |
|---|---|
| Docosahexaensäure (DHA) | 0,5 g - 5 g oder 2,5 g |

**c)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| Docosahexaensäure (DHA) | 0,5 g - 5 g oder 2,5 g |

**d)** Eine Zusammensetzung umfassend die folgenden Bestandteile (Angaben bezogen auf 100 mL sofern nicht anders angegeben) wurde hergestellt:

| | |
|---|---|
| Hochaufgereingtes Fischöl | 5g - 50 g oder 25 g |

Als Hilfsstoffe werden Natriumoleat, Natriumhydroxid und zur Injektion geeignetes Wasser (*aqua ad injectionem*) verwendet.

**Beispiel 2:** Herstellung von Zusammensetzung umfassend Eicosapentaensäure (EPA) und/oder Docosahexaensäure (DHA) mit Beimischungen (MCT, Eisen).

**a)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| MCT | 5 g - 50 g oder 25 g |

**b)** Eine Zusammensetzung umfassend die folgenden Bestandteile (Angaben bezogen auf 100 mL sofern nicht anders angegeben) wurde hergestellt:

| | |
|---|---|
| Docosahexaensäure (DHA) | 0,5 g - 5 g oder 2,5 g |
| MCT | 5 g - 50 g oder 25 g |

**c)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| Docosahexaensäure (DHA) | 0,5 g - 5 g oder 2,5 g |
| MCT | 5 g - 50 g oder 25 g |

**d)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| Eisen | 0,1 mg - 5 mg oder 2,5 mg |

**e)** Eine Zusammensetzung umfassend die folgenden Bestandteile (Angaben bezogen auf 100 mL sofern nicht anders angegeben) wurde hergestellt:

| | |
|---|---|
| Docosahexaensäure (DHA) | 0,5 g - 5 g oder 2,5 g |
| Eisen | 0,1 mg - 5 mg oder 2,5 mg |

**f)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| Docosahexaensäure (DHA) | 0,5 g - 5 g oder 2,5 g |
| Eisen | 0,1 mg - 5 mg oder 2,5 mg |

**g)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| MCT | 5 g - 50 g oder 25 g |
| Eisen | 0,1 mg - 5 mg oder 2,5 mg |

**h)** Eine Zusammensetzung umfassend die folgenden Bestandteile (Angaben bezogen auf 100 mL sofern nicht anders angegeben) wurde hergestellt:

| | |
|---|---|
| Docosahexaensäure (DHA) | 0,5 g - 5 g oder 2,5 g |
| MCT | 5 g - 50 g oder 25 g |
| Eisen | 0,1 mg - 5 mg oder 2,5 mg |

**i)** Eine Zusammensetzung umfassend die folgenden Bestandteile (Angaben bezogen auf 100 mL sofern nicht anders angegeben) wurde hergestellt:

| | |
|---|---|
| Eicosapentaensäure (EPA) | 0,5 g - 5 g oder 2,5 g |
| Docosahexaensäure (DHA) | 0,5 g - 5 g oder 2,5 g |
| MCT | 5 g - 50 g oder 25 g |
| Eisen | 0,1 mg - 5 mg oder 2,5 mg |

14

**j)** Eine Zusammensetzung umfassend die folgenden Bestandteile (Angaben bezogen auf 100 mL sofern nicht anders angegeben) wurde hergestellt:

| | |
|---|---|
| Hochaufgereingtes Fischöl | 5g - 50 g oder 25 g |
| MCT | 5 g - 50 g oder 25 g |

**k)** Eine Zusammensetzung umfassend die folgenden Bestandteile (Angaben bezogen auf 100 mL sofern nicht anders angegeben) wurde hergestellt:

| | |
|---|---|
| Hochaufgereingtes Fischöl | 5g - 50 g oder 25 g |
| Eisen | 0,1 mg - 5 mg oder 2,5 mg |

**l)** Eine Zusammensetzung umfassend die folgenden Bestandteile (Angaben bezogen auf 100 mL sofern nicht anders angegeben) wurde hergestellt:

| | |
|---|---|
| Hochaufgereingtes Fischöl | 5g - 50 g oder 25 g |
| MCT | 5 g - 50 g oder 25 g |
| Eisen | 0,1 mg - 5 mg oder 2,5 mg |

**Beispiel 3:** Herstellung von Omegaven®.

Eine Emulsion (Omegaven®), umfassend die folgenden Bestandteile (Angaben bezogen auf 100 mL sofern nicht anders angegeben) wurde hergestellt:

| | |
|---|---|
| Hochaufgereingtes Fischöl enthaltend: | 10,0g |
| Eicosapentaensäure (EPA) | 1,25g - 2,82g |
| Docosahexaensäure (DHA) | 1,44g - 3,09g |
| Myristinsäure | 0,1g - 0,6g |
| Palmitinsäure | 0,25g - 1,0g |
| Palmitoleinsäure | 0,3g - 0,9g |
| Stearinsäure | 0,05g - 0,2g |
| Ölsäure | 0,6g - 1,3g |
| Linolsäure | 0,1g - 0,7g |
| Linolensäure | $\leq$ 0,2g |
| Octadecatetraensäure | 0,05g - 0,65g |
| Eicosaensäure | 0,05g - 0,3g |
| Arachidonsäure | 0,1g - 0,4g |
| Docosaensäure | $\leq$ 0,15g |
| Docosapentaensäure | 0,15g - 0,45g |
| d1-$\alpha$-Tocopherol | 0,015g - 0,0296g |
| Glycerol | 2,5g |
| gereinigte Phosphatide aus Ei | 1,2g |
| Gesamtenergie | 470kJ/100 mL; 112 kcal/100 mL |
| pH-Wert | 7,5 - 8,5 |
| Titrationssäure | < 1 mmol HCl/L |
| Osmolarität | 308 - 367 mosm/kg |

Als Hilfsstoffe werden Natriumoleat, Natriumhydroxid und zur Injektion geeignetes Wasser (*aqua ad injectionem*) verwendet.

**Beispiel 4:** Intravenöse Verabreichung einer Zusammensetzung umfassend EPA und DHA.

Eine konsekutive intravenöse Verabreichung von Omegaven® erfolgt zwei Tage und einen Tag vor dem Beginn eines Zyklus einer 5-FU basierten adjuvanten Chemotherapie bei einem Patienten mit kolorektalem Karzinom. Das bei dem Patienten angewandte Chemotherapieschema ist das sogenannte FOLFOX-Schema. Die Gabe von Omegaven® erfolgt in einer Dosierung von 2 mL / kg Körpergewicht des Patienten und Tag (siehe auch Abbildungen 1 und 3) Die intravenöse Verabreichung von Omegaven® erfolgt jeweils vor dem Beginn eines Zyklus einer 12 Zyklen umfassenden Chemotherapie. Omegaven® wird vor jedem der 12 Zyklen verabreicht. Auf jeden Zyklus folgt eine mehrtägige Behandlungspause, in der der Patient keine Chemotherapeutika erhält, und die bis zum Beginn des nächsten Zyklus der Chemotherapie reicht. Omegaven® wird jeweils vor dem Beginn eines Zyklus, also während der Behandlungspause, verabreicht.

Eine konsekutive intravenöse Verabreichung von Omegaven® erfolgt zwei Tage, einen Tag und am Tag des Beginns eines Zyklus einer 5-FU basierten adjuvanten Chemotherapie nach dem FOLFOX-Schema bei einem Patienten mit kolorektalem Karzinom. Die Verabreichung am Tag des Beginns des Chemotherapiezyklus erfolgt 3 Stunden vor dem Beginn des Zyklus, und ist spätestens eine Stunde vor dem Beginn des Zyklus beendet. Die Gabe von Omegaven® erfolgt in einer Dosierung von 2 mL / kg Körpergewicht des Patienten und Tag (siehe auch Abbildung 2).

**Beispiel 5:** Untersuchung des Einflusses einer Zusammensetzung umfassend EPA und DHA auf die Wirksamkeit der Chemotherapie und auf chemotherapieinduzierte Nebenwirkungen in einem Mausmodell des humanen kolorektalen Karzinoms.

Für das Experiment werden weibliche NMRI nu/nu Mäuse (Elevage Janvier, Le Genest St Isle, Frankreich) im Alter von 7 bis 8 Wochen mit einem Gewicht von etwa 25 g verwendet. Die Tiere werden nach Erhalt mit $2 \times 10^6$ Zellen / 0,1 mL der humanen kolorektalen Karzinomzelllinie LS174T injiziert, und für zwei Wochen unter standardisierten, spezifiziert pathogenfreien Bedingungen in Isolatoren gehalten, es werden autoklaviertes Einstreu und Futter verwendet.

Zwei Wochen nach der Inokulation mit Tumorzellen werden Mäuse mit Tumoren von etwa 30 mm$^3$ in die verschiedenen experimentellen Gruppen aufgeteilt. Die Aufteilung erfolgt so, dass zwischen den Gruppen vergleichbare mittlere Tumorvolumina vorliegen.

Tiere der Gruppe A erhalten 48 Stunden und 24 Stunden vor Beginn einer Behandlung mit 5-FU jeweils eine intravenöse Infusion mit Omegaven® (2 mL / kg Körpergewicht und Tag; entsprechend 60 μL Omegaven® / 200 μL Saline bei einem Gewicht von 30g). Die Injektion erfolgt über die Schwanzvene. Tiere der Gruppe B erhalten zusätzlich zu der Infusion mit Omegaven® 48 Stunden und 24 Stunden drei Stunden vor Beginn der Behandlung mit 5-FU eine weitere Infusion mit Omegaven®. Tiere der Kontrollgruppe C erhalten 48 Stunden und 24 Stunden vor Beginn der Behandlung mit 5-FU jeweils eine intravenöse Infusion mit Lipovenös® in entsprechender Dosierung. Tiere der Kontrollgruppe D erhalten 24 Stunden und 48 Stunden nach Beginn der Behandlung mit 5-FU jeweils eine intravenöse Infusion mit Omegaven® (2 mL / kg Körpergewicht und Tag). Die Behandlung mit 5-FU (50 mg / kg Körpergewicht und Tag) erfolgt durch intraperetoneale Injektion an sieben aufeinanderfolgenden Tagen.

Einfluss auf die Wirksamkeit der Chemotherapie:

Das Tumorwachstum wird durch die Messung der drei orthogonalen Tumordurchmesser mit einem Messschieber anhand der Formel

$$\text{Tumorvolumen} = 4\pi/3 \text{ x (Länge/ 2x Breite / 2 x Höhe / 2)}$$

bestimmt. Die Messung erfolgt alle zwei Tage. Änderungen des Tumorvolumens werden im Bezug auf das initiale Tumorvolumen eines Tieres berechnet.

Es zeigt sich, dass Tiere der Gruppe A und B gegenüber Tieren der Gruppe C und D ein geringeres mittleres Tumorvolumen aufweisen. Ein Trend zu einem verringerten Tumorvolumen der Tiere der Gruppe B gegenüber der Gruppe A wird beobachtet.

Zusätzlich wird am Ende des Versuchs in einem Teil der Tiere die Einlagerung von [125I] in verschiedenen Geweben durch Untersuchung der Verteilung der Radioaktivität nach zweitägiger Vorbehandlung mit Kaliumjodid und Perchlorat im Trinkwasser (zur Hemmung der Aufnahme von radioaktivem Jod in Thymus und Magen) und einmaliger intravenöser Injektion mit 250 KBq [125I] bestimmt. 24 Stunden nach der Injektion werden die Tiere durch $CO_2$-Inhalation getötet und die Radioaktivität im Tumor und in den verschiedenen Organen (Lunge, Herz, Leber, Magen, Dünndarm, Dickdarm, Milz, Nieren) im γ-Counter (Cobra QC 5002, Packard, US) gemessen. Die Organgewichte werden erfasst.

Anhand des Vergleichs der beobachteten mittlere Einlagerung von [125I] in den Tumoren der Tiere der verschiedenen Gruppen wird ersichtlich, dass eine Verabreichung von Omegaven vor dem Beginn der Chemotherapie (Gruppen A, B) vorteilhaft ist.

Einfluss auf die Nebenwirkungen der Chemotherapie:

Zur Untersuchung des Einflusses einer EPA- und DHA-haltigen Zusammensetzung (Omegaven®) auf die bei der Chemotherapie mit 5-FU auftretenden Nebenwirkungen werden von einem Teil der wie vorstehend beschrieben behandelten Tiere am Ende der Behandlung mit 5-FU verschiedene Parameter erfasst, darunter das Lebergewicht, die $PEG_2$-Aktivität in Leberhomogenaten (Bicyclo-PEG2 Enzym Immunoassay Kit; Cayman Chemical, Ann Abor, MI, USA), sowie das Ausmaß Veränderungen im Darmtrakt der Tiere (Untersuchung von Kryptenhöhe und apoptotischen Figuren in formalinfixierten, in Paraffin eingebetteten histologischen Präparaten nach Hämalaun- und Eosin Färbung), und Veränderungen im Blutbild (Bestimmung der Zellzahlen der einzelnen zellulären Blutbestandteile am Cell-Dyn 3500R).

Das Lebergewicht der Tiere der Gruppen A und B ist höher als das der Tiere der Gruppe C, und die Tiere weisen einen geringeren relativen Gehalt an $PGE_2$ auf. Gegenüber der Kontrollgruppe C treten bei Tieren der Gruppen A und B weniger Veränderungen der Krypten- und Zellstruktur der Normalschleimhaut des Darms auf. Die Tiere der Gruppen A und B haben ein vorteilhafteres, näher an den Normalwerten befindliches Blutbild als Tiere der Kontrollgruppe C.

**Beispiel 6:** Untersuchung des Einflusses von EPA und DHA auf die Bestrahlungssensitivität humaner kolorektaler Zellen *in vitro.*

HT-29 kolorektalen Karzinomzellen (ATCC Nummer HTB-38) wurden in Dulbeccos' modified Eagle's Medium (DMEM, Ref. D 5030, Sigma Chemie AG, Buchs, Schweiz) supplementiert mit 10% hitzeinaktiviertem fötalem Kälberserum (FBS), 1 g/L D-Glucose, 3,7 g/L Natriumbicarbonat und 0,1 g/L Penicillin-Streptomycin (alle Zusätze von Life Technologies Inc., Grand Island, NY) kultiviert. Die Zellen wurden bei 37 °C in befeuchteter Atmosphäre mit 5% $CO_2$ Gehalt kultiviert und durch wöchentlich zweimalige Subkultur in der exponentiellen Wachstumsphase gehalten. Die Kulturen wurden mit Hilfe des MycoAlert Detektionskits (Ref. LT07-118, Cambrex, Verviers, Belgien) auf Mykoplasmeninfektion untersucht.

Der zytotoxische Effekt einer zweitägigen (48 Stunden) Vorbehandlung von HT-29 Zellen mit 0 μM, 20 μM, 50 μM oder 100 μM DHA oder EPA auf eine Bestrahlung der Zellen mit 0 Gy, 2 Gy, 4 Gy oder 6 Gy wurde in einem 15-tägigen Klonogenitätsversuch untersucht.

Dazu wurde das Kulturmedium der Zellen durch Medium, das die entsprechenden Konzentrationen (0 μM, 20 μM, 50 μM oder 100 μM) an DHA oder EPA enthielt, ersetzt. Kontrollen wurden in Medium ohne EPA und ohne DHA kultiviert. Die Zellen wurden für 48 Stunden in dem jeweiligen Medium kultiviert, und dann mit einer einzelnen Strahlendosis von 0 Gy (Kontrolle), 2 Gy, 4 Gy oder 6 Gy in einem X-ray 6 MV Gerät bestrahlt. Die Zellen wurden dann mittels Trypsinierung (1x Trypsin - EDTA, Life Technologies Inc., Grand Island, NY) aus dem Kulturgefäß gelöst, resuspendiert und gezählt. Die Zellen wurden daraufhin nach einem Standartprotokoll seriell verdünnt und in einer Zellzahl, die zur Bildung von etwa 200 Kolonien führt, in 100 x 20 mm Zellkulturschalen mit 10 mL Zellkulturmedium ausgesät. Nach 14-tägiger Inkubation bei 37°C wurden die Gefäße mit Phosphatgepufferter Saline (PBS, Life Technologies Inc., Grand Island, NY) gewaschen, fixiert und mit einer 0,5% Kristallviolettlösung in Methanol/Eisessig (3:1, v/v) angefärbt. Die überlebende Zellfraktion wurde in Bezug auf die unbehandelten Kontrollen berechnet (S/S0).

Die Ergebnisse (siehe Abbildung 4) des Versuchs weisen auf einen synergistischen Effekt einer Dosierung von 20 μM bis 100 μM der Omega-3 Fettsäuren DHA und EPA mit einer Bestrahlung von 2 bis 6 Gy hin. (siehe Abbildung 4).

**Beispiel 7:** Untersuchung des Einflusses von EPA und DHA auf die Bestrahlungssensitivität von Karzinomzellinien *in vitro.*

Humane Karzinomzelllinien (HT-29; ATCC Nummer HTB-38) werden in Dulbeccos' modified Eagle's Medium (DMEM, Ref. D 5030, Sigma Chemie AG, Buchs, Schweiz) supplementiert mit 10% hitzeinaktiviertem fötalem Kälberserum (FBS), 1 g/L D-Glucose, 3,7 g/L Natriumbicarbonat und 0,1 g/L Penicillin-Streptomycin (alle Zusätze von Life Technologies Inc., Grand Island, NY) kultiviert. Die Zellen werden bei 37 °C in befeuchteter Atmosphäre mit 5% $CO_2$ Gehalt kultiviert und durch wöchentlich zweimalige Subkultur in der exponentiellen Wachstumsphase gehalten. Die Kulturen werden mit Hilfe des MycoAlert Detektionskits (Ref. LT07-118, Cambrex, Verviers, Belgien) auf Mykoplasmeninfektion untersucht.

Der zytotoxische Effekt einer Behandlung der Zellen mit DHA, EPA, MCT und Eisen auf eine Bestrahlung der Zellen mit 0 Gy, 2 Gy, 4 Gy oder 6 Gy wird in einem 15-tägigen Klonogenitätsversuch untersucht.

Zunächst wird der Einfluss von 0 μM, 20 μM, 50 μM oder 100 μM DHA oder EPA bei Behandlung der Zellen (A) vor der Bestrahlung im Vergleich zur Behandlung der Zellen nach der Bestrahlung (B) ermittelt. Nicht mit EPA oder DHA behandelte Zellen, die ebenso bestrahlt werden, dienen als Kontrolle (C).

Bei den Zellen, die vor der Bestrahlung mit EPA oder DHA behandelt werden (A), wird das Kulturmedium der Zellen durch Medium mit den entsprechenden Konzentrationen (0 μM, 20 μM, 50 μM oder 100 μM) an DHA oder EPA ersetzt. Die Zellen werden für 48 Stunden in dem Medium kultiviert, und dann mit einer einzelnen Strahlendosis von 0 Gy (Kontrolle), 2 Gy, 4 Gy oder 6 Gy in einem X-ray 6 MV Gerät bestrahlt.

Bei Zellen, die nach der Bestrahlung mit EPA oder DHA behandelt werden (B) und bei den Kontrollzellen (C) wird 48 Stunden vor der Bestrahlung ein Medienwechsel durchgeführt, die Zellen erhalten normales Kulturmedium. Nach 48 Stunden die Zellen mit einer einzelnen Strahlendosis von 0 Gy (Kontrolle), 2 Gy, 4 Gy oder 6 Gy in einem X-ray 6 MV Gerät bestrahlt.

Nach der Bestrahlung werden die Zellen dann mittels Trypsinierung (1x Trypsin - EDTA, Life Technologies Inc., Grand Island, NY) aus dem Kulturgefäß gelöst, resuspendiert und gezählt. Die Zellen werden daraufhin nach einem Standartprotokoll seriell verdünnt und in einer Zellzahl, die zur Bildung von etwa 200 Kolonien führt, in 100 x 20 mm Zellkulturschalen mit 10 mL Medium ausgesät. Zellen der Gruppen A und C werden in normalem Kulturmedium ausgesät, Zellen der Gruppe B in Medium mit den entsprechenden Konzentrationen ($0\mu$M, 20 $\mu$M, 50$\mu$M oder 100$\mu$M) an DHA oder EPA.

Nach 48 Stunden wird bei allen Zellen ein Medienwechsel durchgeführt, nach dem alle Zellen in normalem Kulturmedium kultiviert werden.

Nach insgesamt 14-tägiger Inkubation bei 37°C werden die Gefäße mit Phosphatgepufferter Saline (PBS, Life Technologies Inc., Grand Island, NY) gewaschen, fixiert und mit einer 0,5% Kristallviolettlösung in Methanol/Eisessig (3:1, v/v) angefärbt. Die überlebende Zellfraktion wird in Bezug auf die unbehandelten Kontrollen berechnet (S/S0).

Es zeigt sich, dass bei einer Behandlung mit EPA oder DHA vor der Bestrahlung (A) weniger Kolonien im Bezug auf die unbehandelten Kontrollen (C) und auf die nach der Bestrahlung behandelten Zellen (B) gebildet werden, beziehungsweise dass die gebildeten Kolonien kleiner sind. Die überlebende Zellfraktion ist ebenso geringer.

Der Versuch wird analog mit weiteren Tumorzelllinien (kolorektale Karzinomzelllinien, Mamakarzinomzelllinien und Lungenkarzinomzelllinien) durchgeführt. Auch bei diesen Zelllinien zeigt sich ein stärkerer zytotoxischer Effekt einer Vorbehandlung mit EPA oder DHA im Vergleich zur Nachbehandlung mit EPA oder DHA.

**Beispiel 8:** Einfluss einer Verabreichung von Omegaven® auf Wirksamkeit und Nebenwirkungen einer Bestrahlung *in vivo*

Im ersten Teil des Experiments wird der Einfluss von Omegaven® auf die Wirksamkeit einer Bestrahlung in weiblichen Balb/c Nacktmäusen (Elevage Janvier, Le Genest St Isle, Frankreich) untersucht. Es werden 6 bis 8 Wochen alte Tiere mit einem Gewicht von etwa 25 g verwendet. Die Tiere werden nach Erhalt subkutan mit $4 \times 10^6$ Zellen / 0,1 mL der humanen kolorektalen Karzinomzelllinie HT29 injiziert, und für zwei Wochen unter standardisierten, spezifiziert pathogenfreien Bedingungen in Isolatoren gehalten, es werden autoklaviertes Einstreu und Futter verwendet.

Nach der Inokulation mit Tumorzellen und dem Anwachsen der Tumoren werden die Mäuse in die verschiedenen experimentellen Gruppen aufgeteilt. Die Aufteilung erfolgt so, dass zwischen den Gruppen vergleichbare mittlere Tumorvolumina vorliegen. Gruppe A erhält 48 und 24 vor Beginn der Bestrahlung jeweils eine intravenöse Infusion mit Omegaven® (2 mL / kg Körpergewicht und Tag; entsprechend $60\mu$L Omegaven® / $200\mu$L Saline bei einem Gewicht von 30g). Die Injektion erfolgt über die Schwanzvene. Tiere der Gruppe B erhalten zusätzlich zu der Infusion mit Omegaven® 48 Stunden und 24 Stunden drei Stunden vor Beginn der Bestrahlung eine weitere Infusion mit Omegaven®. Tiere der Kontrollgruppe C erhalten 48 Stunden und 24 Stunden vor Beginn der Bestrahlung jeweils eine intravenöse Infusion mit Lipovenös® in entsprechender Dosierung. Tiere der Kontrollgruppe D erhalten 24 Stunden und 48 Stunden nach Beginn der Bestrahlung jeweils eine intravenöse Infusion mit Omegaven® (2 mL / kg Körpergewicht und Tag). Die Bestrahlung der Tumoren erfolgt durch zweimalige Bestrahlung mit 7,5 Gy.

Einfluss auf die Wirksamkeit der Bestrahlung:

Das Tumorwachstum wird durch die Messung der drei orthogonalen Tumordurchmesser mit einem Messschieber anhand der Formel

$$\text{Tumorvolumen} = 4\pi/3 \text{ x (Länge/ 2x Breite / 2 x Höhe / 2)}$$

bestimmt. Die Messung erfolgt alle zwei Tage. Änderungen des Tumorvolumens werden im Bezug auf das initiale Tumorvolumen eines Tieres berechnet.

Es zeigt sich, dass Tiere der Gruppe A und B gegenüber Tieren der Gruppe C und D ein geringeres mittleres Tumorvolumen aufweisen. Ein Trend zu einem verringerten Tumorvolumen der Tiere der Gruppe B gegenüber der Gruppe A wird beobachtet.

Im zweiten Teil des Experiments wird der Einfluss von Omegaven® auf die Nebenwirkungen der Bestrahlung untersucht. Die verwendeten weiblichen C57 black Mäuse werden wie vorstehend beschrieben gehalten. Die Tiere der Gruppe A erhalten 48 und 24 vor Beginn der Bestrahlung jeweils eine intravenöse Infusion mit Omegaven® (2 mL / kg Körpergewicht und Tag; entsprechend 60μL Omegaven® / 200μL Saline bei einem Gewicht von 30g). Die Injektion erfolgt über die Schwanzvene. Tiere der Gruppe B erhalten zusätzlich zu der Infusion mit Omegaven® 48 Stunden und 24 Stunden drei Stunden vor Beginn der Bestrahlung eine weitere Infusion mit Omegaven®. Tiere der Kontrollgruppe C erhalten 48 Stunden und 24 Stunden vor Beginn der Bestrahlung jeweils eine intravenöse Infusion mit Lipovenös® in entsprechender Dosierung. Tiere der Kontrollgruppe D erhalten 24 Stunden und 48 Stunden nach Beginn der Bestrahlung jeweils eine intravenöse Infusion mit Omegaven® (2 mL / kg Körpergewicht und Tag).Die Bestrahlung der Tiere erfolgt mit einer Einzeldosis von 16,5 Gy. Dabei sind die Tiere durch Bleiplatten so abgeschirmt, dass selektiv die Schnauze der Tiere bestrahlt wird.

Nach der Bestrahlung auftretende Nebenwirkungen (Reaktionen der Epidermis und Mukosa, Ödeme, Gewichtsverlust) werden protokolliert und zwischen den Gruppen verglichen. Die erfassten Parameter umfassen Ausmaß der Schwellung, Ausmaß der Rötung, Schorfbildung.

Es zeigt sich, dass Tiere der Gruppen A und B weniger stark an den Nebenwirkungen der Chemotherapie leiden als Tiere der Gruppe C. Das Ausmaß der Nebenwirkungen ist geringer als das bei den Tieren der Gruppe D beobachtete. Der Versuch zeigt eine protektive Wirkung der Gabe von Omegaven®, der besonders ausgeprägt ist, wenn Omegaven® bereits vor der Bestrahlung verabreicht wurde.

**Patentansprüche**

1. Eine Zusammensetzung umfassend eine Kombination der Omega-3-Fettsäuren Eicosapentaensäure (EPA) und Docosahexaensäure (DHA) zur Verwendung in der Verbesserung der Wirksamkeit einer Chemotherapie oder einer Strahlentherapie und / oder in der Vorbeugung oder Reduktion von durch die Chemotherapie oder die Strahlentherapie hervorgerufenen Nebenwirkungen in einem an Krebs erkrankten Patienten, wobei die Zusammensetzung dem Patienten erst 48 bis 24 Stunden vor Beginn eines Zyklus der Chemotherapie oder der Strahlentherapie parenteral zu verabreichen ist, wobei die Zusammensetzung 1,0 g/ 100 mL bis 7,0 g/100 mL EPA und 1,0 g/ 100 mL bis 7,0 g/100 mL DHA enthält.

2. Die Zusammensetzung zur Verwendung gemäß Anspruch 1, wobei die Nebenwirkungen ausgewählt sind aus der Gruppe bestehend aus gastrointestinale Nebenwirkungen, hämatologische Nebenwirkungen, Reduktion des Lebergewichts, neurotoxische Nebenwirkungen, das Herz betreffende Nebenwirkungen, inflammatorische Nebenwirkungen, Gewichtsverlust, eingeschränkte Funktion der Immunabwehr, Reduktion von Entzündungen oder einer Kombination davon.

3. Die Zusammensetzung zur Verwendung gemäß einem der vorstehenden Ansprüche, wobei die Krebserkrankung ausgewählt ist aus der Gruppe bestehend aus soliden Tumoren und nicht-soliden Tumoren, wobei vorzugsweise die soliden Tumoren ausgewählt sind aus der Gruppe bestehend aus kolorektales Karzinom, Mammakarzinom, Pankreaskarzinom, Leberkarzinom, Lungenkarzinom, und Magenkarzinom.

4. Die Zusammensetzung zur Verwendung gemäß einem der vorstehenden Ansprüche, wobei die Chemotherapie ein Chemotherapeutikum umfasst, das ausgewählt ist aus der Gruppe bestehend aus 5-Fluorouracil, Gemcitabin, Doxorubicin, Paclitaxel, Mitomycin, Cyclophosphamid, Epirubicin, Arabinosylcytosin, Tamoxifen, Irinotecan, Oxaliplatin, Folinsäure, Cisplatin, Taxane, Vinca Alkaloide, Epipodophyllotoxine, synthetische Alkaloide, Cytarabine, Nitrosurea, Dacarbazine, Fludarabine, Ifosfamid, Mytomicin C, Tamoxifen oder einer Kombination davon.

5. Die Zusammensetzung zur Verwendung gemäß einem der vorstehenden Ansprüche, wobei die Chemotherapie 5-Fluorouracil umfasst, wobei vorzugsweise die Chemotherapie FOLFOX oder FOLFIRI ist.

6. Die Zusammensetzung zur Verwendung gemäß einem der vorstehenden Ansprüche, wobei die Strahlentherapie ausgewählt ist aus der Gruppe bestehend aus Teletherapie und Brachytherapie.

7. Die Zusammensetzung zur Verwendung gemäß einem der vorstehenden Ansprüche, wobei die Zusammensetzung weitere bevorzugte Zusatzstoffe ausgewählt aus der Gruppe bestehend aus mittelkettige Fettsäuren und Eisen, oder einer Kombination davon enthält.

8. Die Zusammensetzung zur Verwendung gemäß einem der vorstehenden Ansprüche, wobei die Zusammensetzung 1,0 g/ 100 mL bis 4,0 g/100 mL EPA und 1,0 g/ 100 mL bis 4,0 g/100 mL DHA enthält.

9. Die Zusammensetzung zur Verwendung gemäß einem der vorstehenden Ansprüche, wobei die Zusammensetzung zusätzlich auch noch zwischen 24 und 1 Stunde vor dem Beginn des Zyklus der Chemotherapie oder Strahlentherapie verabreicht wird, wobei vorzugsweise die Zusammensetzung 3 Stunden vor dem Beginn eines Zyklus der Chemotherapie oder der Strahlentherapie verabreicht wird.

10. Die Zusammensetzung zur Verwendung gemäß einem der vorstehenden Ansprüche, wobei die Verabreichung der Zusammensetzung wiederholt wird, wobei vorzugsweise die Zusammensetzung 2- bis 5-mal verabreicht wird.

11. Die Zusammensetzung zur Verwendung gemäß einem der vorstehenden Ansprüche, wobei die Zusammensetzung vor jedem Zyklus der Chemotherapie oder Strahlentherapie verabreicht wird.

12. Die Zusammensetzung zur Verwendung gemäß einem der vorstehenden Ansprüche, wobei 5 mg bis 250 mg pro Kilogramm Körpergewicht und pro Tag EPA, wobei vorzugsweise 20 mg bis 80 mg pro Kilogramm Körpergewicht und pro Tag EPA, am meisten bevorzugt wobei 40 mg bis 60 mg pro Kilogramm Körpergewicht und pro Tag EPA verabreicht werden und wobei 5 mg bis 250 mg pro Kilogramm Körpergewicht und pro Tag DHA, vorzugsweise wobei 20 mg bis 80 mg pro Kilogramm Körpergewicht und pro Tag DHA, am meisten bevorzugt wobei 40 mg bis 60 mg pro Kilogramm Körpergewicht und pro Tag DHA verabreicht werden.

## Claims

1. Composition comprising a combination of the omega-3 fatty acids eicosapentaenoic acid (EPA) and docosahexaenoic acid (DHA) for use in improving the efficacy of chemotherapy or radiotherapy and/or in the prevention or reduction of side effects caused by chemotherapy or radiotherapy in a patient suffering from cancer, wherein the composition should be administered parenterally to the patient only 48 to 24 hours prior to commencement of a cycle of chemotherapy or radiotherapy, wherein the composition comprises 1.0 g/100 mL to 7.0 g/100 mL EPA and 1.0 g/100 mL to 7.0 g/100 mL DHA.

2. Composition for use according to Claim 1, wherein the side effects are selected from the group consisting of gastrointestinal side effects, haematological side effects, reduction in liver weight, neurotoxic side effects, side effects affecting the heart, inflammatory side effects, weight loss, limited function of the immune response, reduction of inflammations or a combination thereof.

3. Composition for use according to either of the preceding claims, wherein the cancerous disorder is selected from the group consisting of solid tumours and non-solid tumours, wherein the solid tumours are preferably selected from the group consisting of colorectal carcinoma, mammary carcinoma, pancreatic carcinoma, liver carcinoma, lung carcinoma and gastric carcinoma.

4. Composition for use according to any of the preceding claims, wherein the chemotherapy comprises a chemotherapeutic agent selected from the group consisting of 5-fluorouracil, gemcitabine, doxorubicin, paclitaxel, mitomycin, cyclophosphamide, epirubicin, arabinosylcytosine, tamoxifen, irinotecan, oxaliplatin, folinic acid, cisplatin, taxane, vinca alkaloids, epipodophyllotoxins, synthetic alkaloids, cytarabine, nitrosourea, dacarbazine, fludarabine, ifosfamide, mitomycin C, tamoxifen or a combination thereof.

5. Composition for use according to any of the preceding claims, wherein the chemotherapy comprises 5-fluorouracil in which the chemotherapy is preferably FOLFOX or FOLFIRI.

6. Composition for use according to any of the preceding claims, wherein the radiotherapy is selected from the group consisting of teletherapy and brachytherapy.

7. Composition for use according to any of the preceding claims, wherein the composition comprises further preferred additives selected from the group consisting of medium-chain fatty acids and iron, or a combination thereof.

8. Composition for use according to any of the preceding claims, wherein the composition comprises 1.0 g/100 mL to 4.0 g/100 mL EPA and 1.0 g/100 mL to 4.0 g/100 mL DHA.

9. Composition for use according to any of the preceding claims, wherein the composition is in addition also administered between 24 hours and 1 hour prior to commencement of the cycle of chemotherapy or radiotherapy, wherein the composition is preferably administered 3 hours prior to commencement of a cycle of chemotherapy or radiotherapy.

10. Composition for use according to any of the preceding claims, wherein administration of the composition is repeated, wherein the composition is preferably administered 2 to 5 times.

11. Composition for use according to any of the preceding claims, wherein the composition is administered prior to each cycle of chemotherapy or radiotherapy.

12. Composition for use according to any of the preceding claims, wherein 5 mg to 250 mg of EPA per kilogram bodyweight and per day are administered, wherein preferably 20 mg to 80 mg of EPA per kilogram bodyweight and per day, most preferably wherein 40 mg to 60 mg of EPA per kilogram bodyweight and per day are administered, and wherein 5 mg to 250 mg of DHA per kilogram bodyweight and per day are administered, preferably wherein 20 mg to 80 mg of DHA per kilogram bodyweight and per day, most preferably wherein 40 mg to 60 mg of DHA per kilogram bodyweight and per day are administered.

## Revendications

1. Composition comprenant une combinaison des acides gras oméga 3 acide eicosapentaénoïque (EPA) et acide docosahexaénoïque (DHA), destinée à une utilisation pour l'amélioration de l'efficacité d'une chimiothérapie ou d'une radiothérapie et/ou pour la prévention ou la réduction des effets secondaires causés par la chimiothérapie ou la radiothérapie chez un patient atteint d'un cancer, la composition n'étant administrée par voie parentérale au patient que 48 à 24 heures avant le début d'un cycle de chimiothérapie ou de radiothérapie, la composition contenant 1,0 g/100 ml à 7,0 g/100 ml d'EPA et 1,0 g/100 ml à 7,0 g/100 ml de DHA.

2. Composition destinée à une utilisation selon la revendication 1, dans laquelle les effets secondaires sont choisis dans le groupe constitué par les effets secondaires gastro-intestinaux, les effets secondaires hématologiques, la réduction du poids du foie, les effets secondaires neurotoxiques, les effets secondaires concernant le coeur, les effets secondaires inflammatoires, la perte de poids, la fonction réduite des défenses immunitaires, la réduction des inflammations ou une combinaison d'entre eux.

3. Composition destinée à une utilisation selon l'une quelconque des revendications précédentes, dans laquelle la maladie cancéreuse est choisie dans le groupe constitué par les tumeurs solides et les tumeurs non solides, les tumeurs solides étant de préférence choisies dans le groupe constitué par le cancer colorectal, le cancer du sein, le cancer du pancréas, le cancer du foie, le cancer du poumon et le cancer de l'estomac.

4. Composition destinée à une utilisation selon l'une quelconque des revendications précédentes, dans laquelle la chimiothérapie comprend un agent chimiothérapeutique, qui est choisi dans le groupe constitué par le 5-fluorouracil, la gemcitabine, la doxorubicine, le paclitaxel, la mitomycine, le cyclophosphamide, l'épirubicine, l'arabinosylcytosine, le tamoxifène, l'irinotécan, l'oxaliplatine, l'acide folique, le cisplatine, les taxanes, les vincaalcaloïdes, les épipodophyllotoxines, les alcaloïdes synthétiques, la cytarabine, la nitrosourée, la dacarbazine, la fludarabine, l'ifosfamide, la mytomicine C, la tamoxifène ou une combinaison d'entre eux.

5. Composition destinée à une utilisation selon l'une quelconque des revendications précédentes, dans laquelle la chimiothérapie comprend du 5-fluorouracil, la chimiothérapie étant de préférence FOLFOX ou FOLFIRI.

6. Composition destinée à une utilisation selon l'une quelconque des revendications précédentes, dans laquelle la radiothérapie est choisie dans le groupe constitué par la téléthérapie et la brachythérapie.

7. Composition destinée à une utilisation selon l'une quelconque des revendications précédentes, dans laquelle la composition contient des additifs préférés supplémentaires choisis dans le groupe constitué par les acides gras à chaînes moyennes et le fer, ou une combinaison d'entre eux.

8. Composition destinée à une utilisation selon l'une quelconque des revendications précédentes, dans laquelle la composition contient 1,0 g/100 ml à 4,0 g/100 ml d'EPA et 1,0 g/100 ml à 4,0 g/100 ml de DHA.

9. Composition destinée à une utilisation selon l'une quelconque des revendications précédentes, dans laquelle la composition est en outre encore administrée entre 24 et 1 heure avant le début du cycle de chimiothérapie ou de radiothérapie, la composition étant de préférence administrée 3 heures avant le début d'un cycle de chimiothérapie ou de radiothérapie.

10. Composition destinée à une utilisation selon l'une quelconque des revendications précédentes, dans laquelle l'administration de la composition est répétée, la composition étant de préférence administrée 2 à 5 fois.

11. Composition destinée à une utilisation selon l'une quelconque des revendications précédentes, dans laquelle la composition est administrée avant chaque cycle de chimiothérapie ou de radiothérapie.

12. Composition destinée à une utilisation selon l'une quelconque des revendications précédentes, dans laquelle 5 mg à 250 mg par kilogramme de poids corporel et par jour d'EPA, de préférence 20 mg à 80 mg par kilogramme de poids corporel et par jour d'EPA, de manière préférée entre toutes 40 mg à 60 mg par kilogramme de poids corporel et par jour d'EPA sont administrés, et dans laquelle 5 mg à 250 mg par kilogramme de poids corporel et par jour de DHA, de préférence 20 mg à 80 mg par kilogramme de poids corporel et par jour de DHA, de manière préférée entre toutes 40 mg à 60 mg par kilogramme de poids corporel et par jour de DHA sont administrés.

**Abbildung 1:**

**Abbildung 2:**

**Abbildung 3:**

Abbildung 4:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9809621 A

**In der Beschreibung aufgeführte Nicht-Patentliteratur**

- **JEMAL et al.** *CA Cancer J Clin,* 2009, vol. 59 (4), 225-49
- **LADEWSKI et al.** *J Clin Oncol.,* 2003, vol. 21 (20), 3859-66
- *Nutrition,* 2001, vol. 17 (6), 478-479